# EUROPÄISCHE PATENTSCHRIFT

(11) **EP 1 765 298 B1**
(45) Veröffentlichungstag und Bekanntmachung des Hinweises auf die Patenterteilung: **24.10.2012**
(21) Anmeldenummer: 05757777.7
(22) Anmeldetag: 29.06.2005
(51) Int. Cl.: A61K 9/14

(54) **VERFAHREN ZUR HERSTELLUNG EINER GEGEN MISSBRAUCH GESICHERTEN, FESTEN DARREICHUNGSFORM UNTER VERWENDUNG EINES PLANETWALZENEXTRUDERS**
METHOD FOR PRODUCING A SOLID DOSAGE FORM, WHICH IS SAFEGUARDED AGAINST ABUSE, WHILE USING A PLANETARY GEAR EXTRUDER
PROCEDE DE PRODUCTION D'UNE FORME POSOLOGIQUE SOLIDE ANTI-ABUS AU MOYEN D'UNE EXTRUDEUSE A VIS PLANETAIRES

(30) Priorität: 01.07.2004 DE 102004032051; 14.07.2004 US 890704
(43) Veröffentlichungstag der Anmeldung: 28.03.2007
(73) Patentinhaber: GRUENENTHAL GMBH, 52078 Aachen (DE)
(72) Erfinder: ARKENAU, Elisabeth, 50931 Köln (DE); BARTHOLOMÄUS, Johannes, 52080 Aachen (DE); KUGELMANN, Heinrich, 52068 Aachen (DE)
(74) Vertreter: Kutzenberger, Helga
(86) Internationale Anmeldenummer: PCT/EP2005/006983
(87) Internationale Veröffentlichungsnummer: WO 2006/002883

(56) Entgegenhaltungen:
- WO-A-2004/037259
- DE-A1- 19 855 440
- US-A1- 2003 044 464
- US-A1- 2003 124 185
- US-B1- 6 228 863
- SCHROEDER R ET AL: "GRANULIERUNG HYDROPHOBER WIRKSTOFFE IM PLANETWALZENEXTRUDER GRANULATION OF HYDROPHOBIC ACTIVES WITH THE PLANETARY ROLLER EXTRUDER" PHARMAZEUTISCHE INDUSTRIE, AULENDORF, DE, Bd. 65, Nr. 4, 2003, Seiten 367-372, XP009012058 ISSN: 0031-711X

## Beschreibung

Die vorliegende Erfindung betrifft ein Verfahren zur Herstellung einer gegen Missbrauch gesicherten, festen Darreichungsform enthaltend wenigstens einen Wirkstoff mit Missbrauchspotential und wenigstens ein synthetisches oder natürliches Polymer (C), mit einer Bruchfestigkeit von ≥ 500 N, dadurch gekennzeichnet, dass eine Mischung umfassend den Wirkstoff und die Polymerkomponente (C) mit Hilfe eines Planetwalzen-Extruders durch Schmelzextrusion zu Formlingen verarbeitet, die ggf. einer Vereinzelung und ggf. einer weiteren Gestaltung zur Darreichungsform unterworfen werden.

Eine Vielzahl von pharmazeutischen Wirkstoffen weist neben einer ausgezeichneten Wirksamkeit auf ihrem betreffenden Anwendungsgebiet auch ein Missbrauchspotential auf, d.h. sie können von einem Missbraucher eingesetzt werden, um Wirkungen herbeizuführen, die nicht ihrem Bestimmungszweck entsprechen.
So werden beispielsweise Opioide, die eine exzellente Wirksamkeit bei der Bekämpfung von starken bis sehr starken Schmerzen zeigen, von Missbrauchern häufig zum Einleiten rauschartiger, euphorisierender Zustände verwendet.

Um Missbrauch zu ermöglichen, werden die entsprechenden Darreichungsformen wie Tabletten oder Kapseln vom Missbraucher zerkleinert, z. B. gemörsert, der Wirkstoff aus dem so erhaltenen Pulver mit Hilfe einer vorzugsweise wäßrigen Flüssigkeit extrahiert und die resultierende Lösung, ggf. nach Filtration durch Watte oder Zellstoff, parenteral, insbesondere intravenös, appliziert. Bei dieser Art der Verabreichung kommt es zu einem gegenüber dem oralen Missbrauch noch zusätzlich beschleunigten Anfluten des Wirkstoffes mit dem vom Missbraucher gewünschten Ergebnis, nämlich den sogenannten Kick. Dieser Kick wird auch erreicht, wenn die gepulverte Darreichungsform nasal missbräuchlich appliziert, d. h. geschnupft wird. Da retardierte Darreichungsformen, die Wirkstoffe mit Missbrauchspotential enthalten, üblicherweise selbst bei einer oralen Einnahme von missbräuchlich hohen Mengen nicht zu dem vom Missbraucher gewünschten Kick führen, werden auch diese zum Missbrauch zerkleinert und extrahiert.

Zur Verhinderung des Missbrauchs wurde in dem US-A- 4,070,494 vorgeschlagen, der Darreichungsform ein quellbares Mittel zuzusetzen. Dieses quillt bei der Zugabe von Wasser zur Extraktion des Wirkstoffes auf und bewirkt, dass das vom Gel separierte Filtrat nur eine geringe Menge Wirkstoff enthält.

Ein entsprechender Ansatz zur Verhinderung des parenteralen Missbrauchs liegt auch der in der WO 95/20947 offenbarten Mehrschichttablette zugrunde, die den Wirkstoff mit Missbrauchspotential und mindestens einen Gelbildner jeweils in unterschiedlichen Schichten getrennt aufweist.

Ein weiterer Ansatz zur Verhinderung des parenteralen Missbrauchs wird in der WO 03/015531 A2 offenbart. Dort wird eine Darreichungsform enthaltend ein analgetisches Opioid und einen Farbstoff als aversives Mittel beschrieben. Die Farbe, die durch unzulässige Manipulation der Darreichungsform freigesetzt wird, soll der Missbraucher davon abhalten, diese manipulierte Darreichungsform zu verwenden.

Eine weitere bekannte Möglichkeit zur Erschwerung des Missbrauchs besteht darin, der Darreichungsform Antagonisten der Wirkstoffe, wie z. B. Naloxon oder Naltexon für Opioide, oder Verbindungen, die zu physiologischen Abwehrreaktionen führen, wie z. B. Raolix Ipecacuama = Brechwurz, der Darreichungsform zuzusetzen (siehe WO 2004/037259).

Da aber nach wie vor in den meisten Fällen für den Missbrauch eine Pulverisierung der Darreichungsform notwendig ist, war es Aufgabe der vorliegenden Erfindung, ein Verfahren zur Herstellung von Darreichungsformen enthaltend Wirkstoffe mit Missbrauchspotential zur Verfügung zu stellen, die bei bestimmungsgemäßer Applikation die gewünschte therapeutische Wirkung gewährleisten, aus denen aber die Wirkstoffe nicht durch einfaches Pulverisieren in eine zum Missbrauch geeignete Form übergeführt werden können.

Diese Aufgabe wurde durch die Bereitstellung des erfindungsgemäßen Verfahren zur Herstellung einer gegen Missbrauch gesicherten, festen Darreichungsform enthaltend wenigstens einen Wirkstoff mit Missbrauchspotential und wenigstens ein synthetisches und/oder natürliches Polymer (C), mit einer Bruchfestigkeit von mindestens 500 N gelöst, das dadurch gekennzeichnet ist, dass eine Mischung umfassend den Wirkstoff und die Polymerkomponente (C) mit Hilfe eines Planetwalzen-Extruders durch Schmelzextrusion zu Formlingen verarbeitet wird, die ggf. einer Vereinzelung und Gestaltung zur Darreichungsform unterworfen werden.

Mit Hilfe des erfindungsgemäßen Verfahrens unter Einsatz der Komponente (C) mit der angegebenen Mindestbruchfestigkeit und der Formulierung mit Hilfe eines Planetwalzen-Extruders gelingt es, auch eine Darreichungsform mit einer Bruchfestigkeit von mindestens 500 N, vorzugsweise mindestens 750 N, zur Verfügung zu stellen, die ein Pulverisieren der Darreichungsform mit üblichen Mitteln und einen darauf folgenden Missbrauch erheblich erschwert bzw. verhindert.

Vorzugsweise liegt die Komponente (C) in solchen Mengen vor, dass die erfindungsgemäß erhaltene Darreichungsform eine Bruchfestigkeit von mindestens 500 N, vorzugsweise 750 N, aufweist.

Wie bereits ausgeführt, ist ohne eine ausreichende Zerkleinerung eine parenterale, insbesondere intravenöse, gefahrlose Applikation nicht möglich oder die Extraktion des Wirkstoffes daraus dauert dem Missbraucher zu lange bzw. der Kick bei missbräuchlicher, oraler Einnahme erfolgt nicht, da keine spontane Freisetzung passiert.

Unter einer Zerkleinerung wird erfindungsgemäß die Pulverisierung der Darreichungsform mit üblichen Mitteln verstanden, die einem Missbraucher üblicherweise zur Verfügung stehen, wie z. B. ein Mörser und Pistill, ein Hammer, ein Schlegel oder andere gebräuchliche Mittel zum Pulverisieren unter Krafteinwirkung.

Planetwalzen-Extruder sind bekannt und u. a. ausführlich im Handbuch der Kunststoff-Extrusionstechnik I (1989) "Grundlagen" im Kapitel 1.2 "Klassifizierung von Extrudern" Seiten 4 bis 6 beschrieben.

Außerdem ist der Einsatz von Planetwalzen-Extrudern zur Herstellung von vorzugsweise porösen Granulaten umfassend einen pharmazeutischen Wirkstoff durch Feuchtgranulation aus der WO 03/028698 erkannt. Ein Hinweis auf die erfindungsgemäße Verfahrensweise, die die Herstellung einer gegen Missbrauch gesicherten Darreichungsform umfassend mindestens einen Wirkstoff mit Missbrauchspotential beschreibt, findet sich in dieser Publikation nicht.

Die erfindungsgemäß hergestellten Darreichungsformen mit einer Bruchfestigkeit von mindestens 500 N, durch die weitgehend ein parenteraler, nasaler und/oder oraler Missbrauch von Wirkstoffen mit Missbrauchspotential verhindert wird, werden daher aus dem Stande der Technik nicht nahegelegt.

Wirkstoffe mit Missbrauchspotential, vorzugsweise pharmazeutische Wirkstoffe mit Missbrauchspotential, sind dem Fachmann ebenso wie deren Herstellung als auch Dosierung bekannt. Sie können als solche, in Form ihrer entsprechenden Derivate, insbesondere Ester, Ether oder Amide, oder jeweils in Form entsprechender physiologisch verträglicher Verbindungen, insbesondere in Form ihrer Salze oder Solvate, als Racemate, Enantiomere oder Stereoisomere durch das erfindungsgemäße Verfahren gegen Missbrauch gesichert werden.

Das erfindungsgemäße Verfahren eignet sich insbesondere zur Verhinderung des Missbrauchs aus einer festen, vorzugsweise oralen Darreichungsform eines vorzugsweise pharmazeutischen Wirkstoffs, der ausgewählt ist aus der Gruppe umfassen Opioide, Tranquillantien, vorzugsweise Benzodiazepine; Barbiturate, Stimulantien und weitere Betäubungsmittel.

Substanzen, die in die Klasse der Opioide fallen sind dem Fachmann beispielsweise aus "Opioid Analgesics" von Alan F. Casy u. a. Auflage von 1986, inbesondere auch Seite 508 bis 518, Plenum Publishing Corporation, "Analgesics" von H. Buschmann, Auflage von 2002, Seite 171 bis 245, WILEY-VCH und "Ullmann's Encyclopedia of Inustrial Chemistry" von Elmar Fiedrichs und andere, 6. Auflage, Seite 1 bis 53, WILEY-VCH bekannt. Die dort aufgeführten Opioide und deren Metabolite sind besonders bevorzugt.

Ganz besonders eignet sich das erfindungsgemäße Verfahren zur Verhinderung des vom Missbrauchs von Darreichungsformen umfassen wenigstens ein Opioid, Tranquillanz oder ein anderes Betäubungsmittels, das ausgewählt ist aus der Gruppe umfassend N-{1-[2-(4-Ethyl-5-oxo-2-tetrazolin-1-yl)ethyl]-4-methoxymethyl-4-piperidyl}propionanilid (Alfentanil), 5,5-Diallylbarbitursäure (Allobarbital), Allylprodin, Alphaprodin, 8-Chlor-1-methyl-6-phenyl-4*H*-[1,2,4]triazolo[4,3-a][1,4]-benzodiazepin (Alprazolam), 2-Diethylaminopropiophenon (Amfepramon), (±)-α-Methylphenethylamin (Amfetamin), 2-(α-Methylphenethylamino)-2-phenylacetonitril (Amfetaminil), 5-Ethyl-5-isopentylbarbitursäure (Amobarbital), Anileridin, Apocodein, 5,5-Diethylbarbitursäure (Barbital), Benzylmorphin, Bezitramid, 7-Brom-5-(2-pyridyl)-1*H*-1,4-benzodiazepin-2(3*H*)-on (Bromazepam), 2-Brom-4-(2-chlorphenyl)-9-methyl-6*H*-thieno[3,2-*f*][1,2,4]triazolo[4,3-a][1,4]diazepin (Brotizolam), 17-Cyclopropylmethyl-4,5α-epopxy-7α[(S)-1-hydroxy-1,2,2-trimethyl-propyl]-6-methoxy-6,14-*endo-*ethanomorphinan-3-ol (Buprenorphin), 5-Butyl-5-ethylbarbitursäure (Butobarbital), Butorphanol, (7-Chlor-1,3-dihydro-1-methyl-2-oxo-5-phenyl-2*H*-1,4-benzodiazepin-3-yl)-dimethyl-carbamat (Camazepam), (1*S*,2*S*)-2-Amino-1-phenyl-1-propanol (Cathin /D-Norpseudoephedrin), 7-Chlor-*N*-methyl-5-phenyl-3*H*-1,4—benzodiazepin-2-ylamin-4-oxid (Chlordiazepoxid), 7-Clor-1-methyl-5-phenyl-1*H*-1,5-benzodiazepin-2,4(3H,5H)-dion (Clobazam), 5-(2-Chlorphenyl)-7-nitro-1*H*-1,4-benzodiazepin-2(3*H*)-on (Clonazepam), Clonitazen, 7-Chlor-2,3-dihydro-2-oxo-5-phenyl-1*H*-1,4-benzodiazepin-3-carbonsäure (Clorazepat), 5-(2-Chlorphenyl)-7-ethyl-1-methyl-1*H-*thieno[2,3-e][1,4]diazepin-2(3*H*)-on (Clotiazepam), 10-Chlor-11b-(2chlorphenyl)-2,3,7,11b-tetrahydrooxazolo[3,2-*d*][1,4]benzodiazepin-6(5*H*)-on (Cloxazolam), (-)-Methyl-[3β-benzoyloxy-2β(1α*H*,5α*H*)-tropancarboxylat] (Cocain), 4,5α-Epoxy-3-methoxy-17-methyl-7-morphinen-6α-ol (Codein), 5-(1-Cyclohexenyl)-5-ethylbarbitursäure (Cyclobarbital), Cyclorphan, Cyprenorphin, 7-Chlor-5-(2-chlorphenyl)-1*H*-1,4-benzodiazepin-2(3*H*)-on (Delorazepam), Desomorphin, Dextromoramid, (+)-(1-Benzyl-3-d)methylamino-2-methyl-1-phenylpropyl)propionat (Dextropropoxyphen), Dezocin, Diampromid, Diamorphon, 7-Chlor-1-methyl-5-phenyl-1*H*-1,4-benzodiatepin-2(3*H*)-on (Diazepam), 4,5α-Epoxy-3-methoxy-17-methyl-6α-morphinanol (Dihydrocodein), 4,5α-Epoxy-17-methyl-3,6a-morphinandiol (Dihydromorphin), Dimenoxadol, Dimephetamol, Dimethylthiambuten, Dioxaphetylbutyrat, Dipipanon, (6a*R*,10aR)-6,6,9-Trimethyl-3-pentyl-6a,7,8,10a-tetrahydro-6H-benzo[c]chromen-1-ol (Dronabinol), Eptazocin, 8-Chlor-6-phenyl-4*H-*[1,2,4]triazolo[4,3-a][1,4]benzodiazepin (Estazolam), Ethoheptazin, Ethylmethylthiambuten, Ethyl-[7-chlor-5-(2-fluorphenyl)-2,3-dihydro-2-oxo-1*H*-1,4 benzodiazepin-3-carboxylat] (Ethylloflazepat), 4,5α-Epoxy-3-ethoxy-17-methyl-7-morphinen-6α-ol (Ethylmorphin), Etonitazen, 4,5α-Epoxy-7α-(1-hydroxy-1-methylbutyl)-6-methoxy-17-methyl-6,14-*endo*-etheno-morphinan-3-ol (Etorphin), *N-*Ethyl-3-phenyl-8,9,10-trinorboman-2-ylamin (Fencamfamin), 7-[2-(α-Methylphenethylamino)ethyl]-theophyllin) (Fenetyllin), 3-(α-Methylphenethylamino)propionitril (Fenproporex), *N*-(1-Phenethyl-4-piperidyl)propionanilid (Fentanyl), 7-Chlor-5-(2-fluorphenyl)-1-methyl-1*H*-1,4-benzodiazepin-2(3*H*)-on (Fludiazepam), 5-(2-Fluorphenyl)-1-methyl-7-nitro-1H-1,4-benzodiazepin-2(3*H*)-on (Flunitrazepam), 7-Chlor-1-(2-diethylaminoethyl)-5-(2-fluorphenyl)-1*H*-1,4-benzodiazepin-2(3*H*)-on (Flurazepam), 7-Chlor-5-phenyl-1-(2,2,2-trifluorethyl)-1*H*-1,4-benzodiazepin-2(3*H*)-on (Halazepam), 10-Brom-11b-(2-fluorphenyl)-2,3,7,11b-tetrahydro[1,3]oxazolo[3,2-d][1,4]benzodiazepin-6(5*H*)-on (Haloxazolam), Heroin, 4,5α-Epoxy-3-methoxy-17-methyl-6-morphinanon (Hydrocodon), 4,5α-Epoxy-3-hydroxy-17-methyl-6-morphinanon (Hydromorphon), Hydroxypethidin, Isomethadon, Hydroxymethylmorphinan, 11-Chlor-8,12b-dihydro-2,8-dimethyl-12b-phenyl-4*H*-[1,3]oxazino[3,2-d][1,4]benzodiazepin-4,7(6*H*)-dion (Ketazolam), 1-[4-(3-Hydroxyphenyl)-1-methyl-4-piperidyl]-1-propanon (Ketobemidon), (3*S*,6*S*)-6-Dimethylamino-4,4-diphenylheptan-3-ylacetat (Levacetylmethadol (LAAM)), (-)-6-Dimethylamino-4,4-diphenyl-3-heptanon (Levomethadon), (-)-1 7-Methyl-3-morphinanol (Levorphanol), Levophenacylmorphan, Lofentanil, 6-(2-Chlorphenyl)-2-(4-methyl-1-piperazinylmethylen)-8-nitro-2*H-*imidazo[1,2-a][1,4] benzodiazepin-1(4*H*)-on (Loprazolam), 7-Chlor-5-(2-chlorphenyl)-3-hydroxy-1*H*-1,4-benzodiazepin-2(3*H*)-on (Lorazepam), 7-Chlor-5-(2-chlorphenyl)-3-hydroxy-1-methyl-1*H*-1,4-benzodiazepin-2(3*H*)-on (Lormetazepam), 5-(4-Chlorphenyl)-2,5-dihydro-3*H*-imidazo[2,1-*a*]isoindol-5-ol (Mazindol), 7-Chlor-2,3-dihydro-1-methyl-5-phenyl-1*H*-1,4-benzodiazepin (Medazepam), *N*-(3-Chlorpropyl)-α-methylphenethylamin (Mefenorex), Meperidin, 2-Methyl-2-propyltrimethylendicarbamat (Meprobamat), Meptazinol, Metazocin, Methylmorphin, N,α-Dimethylphenethylamin (Metamfetamin), (±)-6-Dimethylamino-4,4-diphenyl-3-heptanon (Methadon), 2-Methyl-3-o-tolyl-4(3*H*)-chinazolinon (Methaqualon), Methyl-[2-phenyl-2-(2-piperidyl)acetat] (Methylphenidat), 5-Ethyl-1-methyl-5-phenylbarbitursäure (Methylphenobarbital), 3,3-Diethyl-5-methyl-2,4-piperidindion (Methyprylon), Metopon, 8-Chlor-6-(2-fluorphenyl)-1-methyl-4*H*-imidazo[1,5-a][1,4]benzodiazepin (Midazolam), 2-(Benzhydrylsulfinyl)acetamid (Modafinil), 4,5α-Epoxy-17-methyl-7-morphinen-3,6a-diol (Morphin), Morphin-6-glucoronid, Myrophin, (±)-*trans*-3-(1,1-Dimethylheptyl)-7,8,10,10α-tetrahydro-1-hydroxy-6,6-dimethyl-6*H-*dibenzo [b, *d*]pyran-9(6α*H*)-on (Nabilon), Nalbuphen, Nalorphin, Narcein, Nicomorphin, 1-Methyl-7-nitro-5-phenyl-1*H*-1,4-benzodiazepin-2(3*H*)-on (Nimetazepam), 7-Nitro-5-phenyl-1*H*-1,4-benzodiazepin-2(3*H*)-on (Nitrazepam), 7-Chlor-5-phenyl-1*H*-1,4-benzodiazepin-2(3*H*)-on (Nordazepam), Norlevorphanol, 6-Dimethylamino-4,4-diphenyl-3-hexanon (Normethadon), Normorphin, Norpipanon, der geronnene Saft der zur Art Papaver somniferum gehörenden Pflanzen (Opium), 7-Chlor-3-hydroxy-5-phenyl-1*H*-1,4-benzodiazepin-2(3*H*-on (Oxazepam), (*cis-trans*)-10-Chlor-2,3,7,11b-tetrahydro-2-methyl-11b-phenyloxazolo[3,2-*d*][1,4] benzodiazepin-6-(5*H*)-on (Oxazolam), 4,5α-Epoxy-14-hydroxy-3-methoxy-17-methyl-6-morphinanon (Oxycodon), Oxymorphon, Pflanzen und Pflanzenteile der zur Art Papaver somniferum (einschließlich der Unterart setigerum) gehörenden Pflanzen (Papaver somniferum), Papaveretum, 2-Imino-5-phenyl-4-oxazolidinon (Pernolin), 1,2,3,4,5,6-Hexahydro-6,11-dimethyl-3-(3-methyl-2-butenyl)-2,6-methano-3-benzazocin-8-ol (Pentazocin), 5-Ethyl-5-(1-methylbutyl)-barbitursäure (Pentobarbital), Ethyl-(1-methyl-4-phenyl-4-piperidincarboxylat) (Pethidin), Phenadoxon, Phenomorphan, Phenazocin, Phenoperidin, Piminodin, Pholcodein, 3-Methyl-2-phenylmorpholin (Phenmetrazin), 5-Ethyl-5-phenylbarbitursäure (Phenobarbital), α,α-Dimethylphenethylamin (Phentermin), 7-Chlor-5-phenyl-1-(2-propinyl)-1*H*-1,4-benzodiazepin-2(3*H*)-on (Pinazepam), α-(2-Piperidyl)benzhydrylalkohol (Pipradrol), 1'-(3-Cyan-3,3-diphenylpropyl)[1,4'-bipiperidin]-4'-carboxamid (Piritramid), 7-Chlor-1-(cyclopropylmethyl)-5-phenyl-1*H*-1,4-benzodiazepin-2(3*H*)-on (Prazepam), Profadol, Proheptazin, Promedol, Properidin, Propoxyphen, N-(1-Methyl-2-piperidinoethyl)-N-(2-pyridyl)propionamid, Methyl{3-[4-methoxycarbonyl-4-(*N-*phenylpropanamido)piperidino]propanoat} (Remifentanil), 5-sec-Butyl-5-ethylbarbitursäure (Secbutabarbital), 5-Allyl-5-(1-methylbutyl)-barbitursäure (Secobarbital), *N*-{4-Methoxymethyl-1-[2-(2-thienyl)ethyl]-4-piperidyl}propionanilid (Sufentanil), 7-Chlor-2-hydroxy-methyl-5-phenyl-1*H*-1,4-benzodiazepin-2(3*H*)-on (Temazepam), 7-Chlor-5-(1-cyclohexenyl)-1-methyl-1*H*-1,4-benzodiazepin-2(3*H*)-on (Tetrazepam), Ethyl-(2-dimethylamino-1-phenyl-3-cyclohexen-1-carboxylat) (Tilidin (cis und trans)), Thebain, Tramadol, 8-Chlor-6-(2-chlorphenyl)-1-methyl-4*H-*[1,2,4]triazolo[4,3-a][1,4]benzodiazepin (Triazolam), 5-(1-Methylbutyl)-5-vinylbarbitursäure (Vinylbital), (1R,2R)-3-(3-Dimethylamino-1-ethyl-2-methyl-propyl)-phenol, (1 R, 2R, 4S)-2- [Dimethylamino)methyl-4-(p-fluorbenzyloxy)-(m-methoxyphenyl)cyclohexanol, (1 R, 2R)-3-(2-Dimethylaminomethyl-cyclohexyl)-phenol, (1 S, 2S)-3(3-Dimethylamino-1-ethyl-2-methyl-propyl)-phenol, (2R, 3R)-1-Dimethylamino-3(3-Methoxy-phenyl)-2-methyl-pentan-3-ol, (1RS, 3RS, 6RS)-6-Dimethylaminomethyl-1-(3-methoxy-phenyl)-cyclohexan-1,3-diol, vorzugsweise als Racemat, 3-(2-Dimethylaminomethyl-1-hydroxy-cyclohexyl)-phenyl 2-(4-isobutylphenyl)-propionat, 3-(2-Dimethylaminomethyl-1-hydroxy-cyclohexyl)phenyl 2-(6-methoxy-naphthalen-2-yl)-propionat, 3-(2-Dimethylaminomethyl-cyclohex-1-enyl)-phenyl 2-(4-isobutyl-phenyl)-propionat, 3-(2-Dimethylaminomethyl-cyclohex-1-enyl)-phenyl 2-(6-methoxy-naphthalen-2-yl)-propionat, (RR-SS)-2-Acetoxy-4-trifluoromethyl-benzoesäure 3-(2-dimethylaminomethyl-1-hydroxy-cyclohexyl)-phenyl ester, (RR-SS)-2-Hydroxy-4-trifluoromethyl-benzoesäure 3-(2-dimethylaminomethyl-1-hydroxy-cyclohexyl)-phenyl ester, (RR-SS)-4-Chloro-2-hydroxy-benzoesäure 3-(2-dimethylaminomethyl-1-hydroxy-cyclohexyl)-phenyl ester, (RR-SS)-2-Hydroxy-4-methyl-benzoesäure 3-(2-dimethylaminomethyl-1-hydroxy-cyclohexyl)-phenyl ester, (RR-SS)-2-Hydroxy-4-methoxy-benzoesäure 3-(2-dimethylaminomethyl-1-hydroxy-cyclohexyl)-phenyl-ester, (RR-SS)-2-Hydroxy-5-nitro-benzoesäure 3-(2-dimethylaminomethyl-1-hydroxy-cyclohexyl)-phenyl ester, (RR-SS)-2',4'-Difluoro-3-hydroxy-biphenyl-4-carbonsäure 3-(2-dimethylaminomethyl-1-hydroxy-cyclohexyl)-phenyl ester sowie entsprechende stereoisomere Verbindungen, jeweils deren entsprechende Derivate, insbesondere Amide, Ester oder Ether, und jeweils deren physiologisch verträgliche Verbindungen, insbesondere deren Salze und Solvate, besonders bevorzugt Hydrochloride Sulfate oder Saccharinate.

Das erfindungsgemäße Verfahren eignet sich insbesondere zur Verhinderung des Missbrauchs eines opioiden Wirkstoffes ausgewählt aus der Gruppe umfassend Oxycodon, Hydromorphon, Morphin, Tramadol und deren physiologisch verträgliche Derivate oder Verbindungen, vorzugsweise deren Salze und Solvate, vorzugsweise deren Hydrochloride, Sulfate oder Saccharinate.

Weiterhin eignet sich das erfindungsgemäße Verfahren insbesondere zur Verhinderung des Missbrauchs eines opioiden Wirkstoffes ausgewählt aus der Gruppe umfassend (1R, 2R)-3-(3-Dimethylamino-1-ethyl-2-methyl-propyl)-phenol, (2R, 3R)-1-Dimethylamino-3-(3-methoxy-phenyl)-2-methyl-pentan-3-ol, (1 RS, 3RS, 6RS)-6-Dimethylaminomethyl-1-(3-methoxy-phenyl)-cyclohexane-1,3-diol, (1R, 2R)-3-(2-Dimethylaminonethyl-cyclohexyl)-phenol, deren physiologisch verträglichen Salze, vorzugsweise Hydrochloride, Sulfate, Saccharinate, deren physiologisch verträgliche Enantiomere, Stereoisomere, Diastereomere und Racemate und deren physiologisch verträglichen Derivate, vorzugsweise Ether, Ester oder Amide.

Diese Verbindung bzw. deren Herstellungsverfahren sind in der EP-A-693475 bzw. EP-A-780369 beschrieben.

Zur Erzielung der notwendigen Bruchfestigkeit werden in dem erfindungsgemäßen Verfahren mindestens ein synthetisches, halbsynthetisches und/oder natürliches Polymer (C) mit einer Bruchfestigkeit, gemessen nach der in der vorliegenden Anmeldung offenbarten Methode, von mindestens 500 N, vorzugsweise 750 N, eingesetzt. Bevorzugt wird hierfür mindestens ein Polymeres ausgewählt aus der Gruppe umfassend Polyalkylenoxide, vorzugsweise Polymethylenoxide, Polyethylenoxide, Polypropylenoxide, Polyolefine, vorzugsweise Polyethylene, Polypropylene, Polyvinylchloride, Polycarbonate, Polystyrole, Poly(meth)acrylate, deren Copolymerisate und Mischungen aus mindestens zwei Vertretern der genannten Polymerklassen oder Polymeren eingesetzt. Besonders bevorzugt wird ein wasserlösliches oder wasserquellbares Polymer eingesetzt. Bevorzugt sind hochmolekulare, thermoplastische Polyalkylenoxide. Besonders bevorzugt sind Polyethylenoxide mit einem Molekulargewicht von mindestens 0,5 Mio., vorzugsweise von mindestens 1 Mio., besonders bevorzugt von 1 Mio. bis 15 Mio., bestimmt durch rheologische Messungen. Diese Polyethylenoxide weisen eine Viskosität bei 25 °C von 4500 bis 17600 cP, gemessen an einer 5 Gew.% wässrigen Lösung des Polymeren mit Hilfe eines Brookfield Viskosimeters, Model RVF (Spindel Nr. 2 / Rotationsgeschwindigkeit 2 rpm), von 400 bis 4000 cP, gemessen an einer 2 Gew.% wässrigen Lösung des Polymeren mit Hilfe des genannten Viskosimeters (aber mit Spindel Nr. 1 bzw. 3 / Rotationsgeschwindigkeit 10 rpm) bzw. von 1650 bis 10000 cP, gemessen an einer 1 Gew.% wässrigen Lösung des Polymers mit Hilfe des genannten Viskosimeters (aber mit Spindel Nr. 2 /Rotationsgeschwindigkeit 2 rpm) auf (vgl. Handbook of Pharmaceutical Excipients von Raymond C. Rowe u. a., Ausgabe 4., 2003, Seite 460).

Die Polymeren werden vorzugsweise als Pulver eingesetzt. Sie können wasserlöslich oder wasserquellbar sein.

Vorzugsweise wird die Komponente (C) in einer Menge von 20 bis 99,9 Gew.%, besonders bevorzugt von wenigstens 35 Gew.%, ganz besonders bevorzugt von wenigstens 50 Gew.%, bezogen auf das Gesamtgewicht der Darreichungsform, eingesetzt.

Als Hilfsstoffe (B) können die üblichen für die Formulierung von festen Darreichungsformen bekannte Hilfsstoffe verwendet werden. Vorzugsweise sind diese Weichmacher, wie Polyethylenglykol in Mengen von 0,01 bis 20 Gew.%, besonders bevorzugt bis 15 Gew.% und ganz besonders bevorzugt bis 10 Gew.%, Hilfsstoffe, die die Wirkstofffreisetzung beeinflussen, wie nachstehend aufgeführt, vorzugsweise hydrophobe oder hydrophile, vorzugsweise hydrophile Polymere, ganz besonders bevorzugt Hydroxypropylmethylcellulose oder Hydroxypropylcellulose, und/oder Antioxidantien. Als Antioxidantien eignen sich Ascorbinsäure, Butylhydroxyanisol, Butylhydroxytoluol, Salze der Ascorbinsäure, Monothioglyzerin, phosphorige Säure, Vitamin C, Vitamin E und dessen Derivate, Natriumbisulfit, besonders bevorzugt Butylhydroxytoluol (BHT) oder Butylhydroxyanisol (BHA) und α-Tocopherol.

Das Antioxidanz wird vorzugsweise in Mengen von 0,01 bis 10 Gew.%, vorzugsweise 0,03 bis 5 Gew.%, bezogen auf das Gesamtgewicht der Darreichungsform, eingesetzt.

Des weiteren können neben den vorstehend genannten Polymeren zusätzlich zur Erzielung der notwendigen Bruchfestigkeit der Darreichungsform in dem erfindungsgemäßen Verfahren mindestens ein natürliches, halbsynthetisches oder synthetisches Wachs (D) mit einer Bruchfestigkeit, gemessen nach der in der vorliegenden Anmeldung offenbarten Methode von mindestens 500 N, vorzugsweise 750 N, mit eingesetzt werden. Bevorzugt sind Wachse mit einem Erweichungspunkt von mindestens 60°C. Besonders bevorzugt sind Carnaubawachs und Bienenwachs. Ganz besonders bevorzugt ist Carnaubawachs. Carnaubawachs ist ein natürliches Wachs, das aus den Blättern der Carnaubapalme gewonnen wird und einen Erweichungspunkt von höchstens 90°C aufweist. Bei einem zusätzlichen Einsatz der Wachskomponente wird diese zusammen mit wenigstens einem Polymeren (C), vorzugsweise mit wenigstens einem Polyethylenoxid, in solchen Mengen eingesetzt, dass die Darreichungsform eine Bruchfestigkeit von mindestens 500 N, vorzugsweise mindestens 750 N, gemessen nach der in der vorliegenden Anmeldung angegebenen Methode, aufweist.

Überraschenderweise wird durch die Extrusion einer Mischung umfassend den Wirkstoff mit Missbrauchspotential (A) und mit Hilfe eines Planetwalzen-Extruders nach dem Schmelzverfahren in einer einfachen und reproduzierbaren Weise die notwendige Mindest-Bruchfestigkeit von mindestens 500 N erreicht, die gewährleistet, dass ein Pulverisieren der Darreichungsform mit üblichen Mitteln nicht möglich ist und damit ein anschließender Missbrauch erheblich erschwert bzw. verhindert wird.

Zur Durchführung des erfindungsgemäßen Verfahrens wird wenigstens ein Wirkstoff mit Missbrauchspotential, die Komponente bzw. die Komponenten (C) und gegebenenfalls ein Wachs (D), gegebenenfalls mindestens eine der nachfolgend aufgeführten gegebenenfalls vorhandenen weiteren missbrauchsverhindernden Komponenten (a) bis (f) und gegebenenfalls vorhandene Hilfsstoffe (B) wie Antioxidantien, Weichmacher und/oder retardierende Hilfsstoffe mit Hilfe eines Planetwalzen-Extruders zu der Darreichungsform verarbeitet.

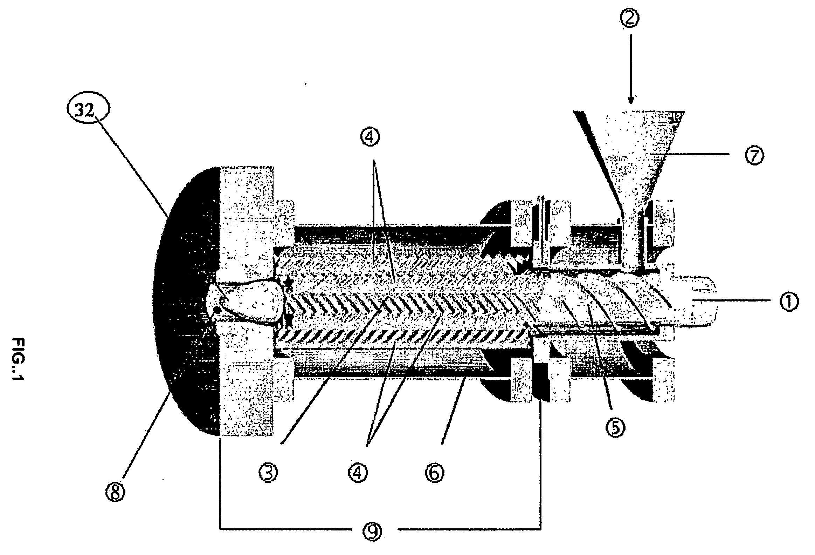

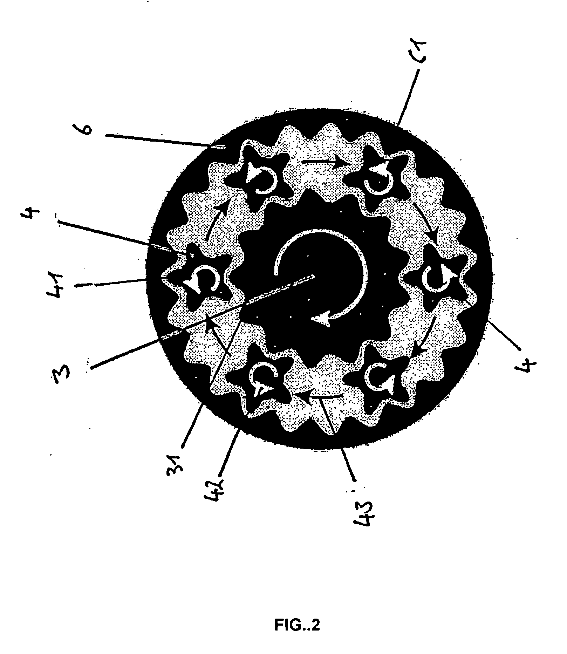

Im folgenden wird der Einsatz eines Planetwalzen-Extruders in dem erfindungsgemäßen Verfahren anhand der **Figuren 1** **und** **2** erläutert. Diese Erläuterungen sind lediglich beispielhaft und schränken den allgemeinen Erfindungsgedanken nicht ein.
- **Figur 1**: zeigt den Schnitt durch einen Planetwalzen-Extruder und
- **Figur 2**: zeigt die Wirkungsweise des Planetwalzen-Extruders.

In **Figur 1** ist ein Planetwalzen-Extruder dargestellt, der gemäß dem erfindungsgemäßen Verfahren eingesetzt werden kann. Dieser Extruder weist im wesentlichen eine Welle 1 auf, die bezogen auf die Transportrichtung der zu extrudierenden Mischung der vorstehend aufgeführten Komponenten zunächst als Einzugsschnecke 5 und im weiteren als Zentralspindel 3 mit einem Spindelkopf im Düsenbereich (32) des Planetwalzen-Extruders gestaltet ist. Um die Zentralspindel 3 sind vorzugsweise drei bis sieben Planetspindeln 4 angeordnet, die wiederum von einem Mantel in Form eines Gehäuses 6 umgeben sind.

Im Planetwalzen-Extruder wird unter Bezugnahme auf **Figur 1** die Extrusion der im erfindungsgemäßen Verfahren zum Einsatz kommenden Zusammensetzung zur Herstellung einer pharmazeutischen Darreichungsform, vorzugsweise wie folgt durchgeführt. Wie durch Pfeil 2 dargestellt, werden die zu extrudierenden Komponenten mittels entsprechender Pulverdosierer, wenigstens ein Dosierer, durch die Einfüllvorrichtung 7 in den Bereich der Einzugsschnecke 5 dosiert und durch deren Drehung (Antrieb nicht dargestellt) in Richtung der Zentralspindel 3 befördert. Der Fachmann versteht, dass im Bereich der Einzugsschnecke ein Vermischen der Ausgangsstoffe (Komponenten) möglich ist. Es ist aber auch möglich, die Komponenten der Darreichungsform vorzumischen und diese Mischung über die Einfüllvorrichtung 7 in dem Bereich der Einzugsschnecke 5 zu dosieren. Im Einzugsbereich des Planetwalzen-Extruders wird die Mischung zur Extrusionsbereich (9) weiterbefördert, wo die mindestens bis zur Erweichung der Komponente (C) erwärmt wird. Im Bereich der Zentralspindel 3, d. h. im Extrusionsbereich 9, wird die erwärmte Mischung durch Zusammenwirkung der Zentralspindel 3 und der Planetspindeln 4 gefördert, ggf. weiter homogenisiert, komprimiert bzw. kompaktiert und zum Düsenbereich 8 gefördert, wo ein Extrusionsstrang oder Extrusionsstränge, je nach dem wie viele Bohrungen die Düse aufweist extrudiert werden. Die Düsengeometrie bzw. die Geometrie der Bohrungen ist frei wählbar. So kann die Düse bzw. die Bohrungen einen runden, oblongen oder ovalen Querschnitt aufweisen, wobei der runde Querschnitt vorzugsweise einen Durchmesser 0,1 mm bis 15 mm, der oblonge bzw. ovale Querschnitt vorzugsweise eine maximale Länge von 21 mm und eine maximale Breite von 10 mm aufweist. Die Extrusionsdüse kann auch als Schlitzdüse gestaltet sein. Vorzugsweise hat die Düse bzw. die Bohrungen einen runden Querschnitt. Sowohl der Mantel 6 des erfindungsgemäß zum Einsatz kommenden Planetwalzen-Extruders als auch die Zentralspindel können beheizt oder gekühlt werden. Die entsprechende Temperierung, d. h. Beheizung oder Kühlung, richtet sich danach, dass die zu extrudierende Mischung mindestens eine Durchschnittstemperatur entsprechend der notwendigen Erweichung der Komponente (C) aufweist und nicht über eine Temperatur steigt, bei der der zur verarbeitende Wirkstoff mit Missbrauchspotential Schaden nehmen kann. Vorzugsweise wird die Temperatur der zu extrudierenden Mischung unter 180°C, vorzugsweise unter 150 °C, aber mindestens auf die Erweichung der Komponente (C), eingestellt. Durch die Komprimierung der zumindest plastifizierten Mischung und Extrusion erhält man Darreichungsformen, die eine Bruchfestigkeit von mindestens 500 N aufweisen.

Nach der Extrusion der zumindest teilweise geschmolzenen Mischung und gegebenenfalls Kühlung des extrudierten Stranges bzw. der extrudierten Stränge erfolgt ggf. eine nicht in Figur 1 dargestellte Zerkleinerung der Extrudate. Diese Zerkleinerung kann vorzugsweise durch Zerschneiden der Extrudate mittels mitlaufender oder rotierender Messer, Wasserstrahlschneider, Drähten, Klingen oder mit Hilfe von Laserschneidern oder Ultraschallschneidern durchgeführt werden.

Gegebenenfalls nach einem weiteren Abkühlen der zerkleinerten Extrudate, die vorzugsweise in Scheiben vorliegen, erfolgt gegebenenfalls ein Umformen in die Endform der Darreichungsform, wobei, wenn nötig, wieder Wärmeeinwirkung erfolgt.

Diese Formgebung beispielsweise zu Tabletten kann dadurch erfolgen, dass das plastische Extrudat mit Hilfe von zwei gegenläufig angetriebenen Walzen mit vorzugsweise zur Plastifizierung einander gegenüberliegenden Vertiefungen im Walzenmantel, deren Ausführung die Tablettenform bestimmt, unter Verpressung geformt wird.

Es ist aber auch möglich, aus den vereinzelten Extrudaten die Tabletten jeweils mit Hilfe einer gegebenenfalls beheizten Matrize und mindestens einem formgebenden Stempel zu formen. Dazu können vorzugsweise die nach der Zerkleinerung des extrudierten Stranges erhaltenen zylinderförmigen Granulate verwendet werden. Außer zu Tabletten verpresst, können diese Granulate oder andere erhaltene multipartikuläre Formen, wie Pellets oder Spheriode, auch in Kapseln abgefüllt werden, um als erfindungsgemäß hergestellte Darreichungsform verwendet zu werden.

In einer weiteren bevorzugten Ausführungsform können die durch mehrere Bohrungen der Extrusionsdüse extrudierten Stränge gegebenenfalls nach deren Abkühlung durch Verflechtung oder Umschlingung entsprechend einer Seilherstellung zu einem gegenüber den einzelnen extrudierten Strängen dickeren Strang zusammengeführt werden. Dieser Strang kann gegebenenfalls durch Anlösen mit geeignetem Lösungsmittel oder durch Erwärmung bis zum Erweichungspunkt der Komponten (C) und gegebenenfalls Entfernen des Lösungsmittels entsprechend der vorstehend aufgeführten Zerkleinerung und Verformung eines einzelnen Stranges weiter verarbeitet werden.

**Figur 2** zeigt eine Querschnitt durch den Planetwalzen-Extruder. Um die sich drehende Zentralspindel 3 sind mindestens drei, in dem gezeigten Fall 6, Planetspindeln 4 angeordnet, deren Flanken 41 zu einem mit dem Flanken 31 der Zentralspindel 3 und zum anderen mit den Flanken 61 des Mantels 6 des Planetwalzen-Extruders zusammenwirken. Durch die Drehung der Zentralspindel 3 und das Abrollen der jeweiligen Flanken aufeinander drehen sich die Planetspindeln 4 jeweils wie mit Pfeil 42 um ihre eigene Achse und wie Pfeil 43 verdeutlicht, um die Zentralspindel 4 herum. Dadurch wird die erfindungsgemäß angestrebte Komprimierung bzw. Kompaktierung der erfindungsgemäß zum Einsatz kommenden Komponentenmischung der erfindungsgemäß hergestellten Darreichungsformen bewirkt.

Sofern notwendig, kann der zum Einsatz kommende Planetwalzen-Extruder nicht nur einen Extrusionsbereich (9) aufweisen, sondern mindestens noch einen weiteren, um gegebenenfalls auch die zu extrudierende Mischung entgasen zu können.

Das erfindungsgemäße Verfahren kann diskontinuierlich oder kontinuierlich, vorzugsweise kontinuierlich, durchgeführt werden.

Die durch den erfindungsgemäßen Verfahren erhaltenen Darreichungsformen zeichnen sich wie erwähnt dadurch aus, dass sie aufgrund ihrer Härte nicht zu pulverisieren sind. Ein oraler, parenteraler, inbesondere intravenöser oder nasaler Missbrauch ist dadurch praktisch ausgeschlossen. Um jedoch jeden möglichen Missbrauch bei einer Zerkleinerung und /oder bei einer dennoch ggf. durch außergewöhnliche Krafteinwirkung auftretende Pulverisierung der durch den erfindungsgemäßen Herstellungsverfahren erhaltenen Darreichungsformen vorzubeugen, können diese Darreichungsformen in einer bevorzugten Ausführungsform als Hilfsstoffe weitere missbrauchs-erschwerende bzw. - verhindernde Mittel enthalten.

So kann die erfindungsgemäß erhaltene Darreichungsform, die neben wenigstens einem Wirkstoff mit Missbrauchspotential, wenigstens eine Polymerkomponente (C) und ggf. ein Wachs (D), noch wenigstens eine der nachfolgenden Komponenten aufweisen:
(a) wenigstens einen den Nasen- und/oder Rachenraum reizenden Stoff,
(b) wenigstens ein viskositätserhöhendes Mittel, das in einem mit Hilfe einer notwendigen Mindestmenge an einer wässrigen Flüssigkeit aus der Darreichungsform gewonnenen Extrakt ein Gel bildet, welches vorzugsweise beim Einbringen in eine weitere Menge einer wässrigen Flüssigkeit visuell unterscheidbar bleibt,
(c) wenigstens einen Antagonisten für jeden der Wirkstoffe mit Missbrauchspotential,
(d) wenigstens ein Emetikum.
(e) wenigstens einen Farbstoff als aversives Mittel
(f) wenigstens einen Bitterstoff

Die Komponenten (a) bis (f) sind jeweils für sich allein zusätzlich zur Sicherung der durch das erfindungsgemäße Verfahren erhaltenen Darreichungsformen gegen Missbrauch geeignet. So eignet sich die Komponente (a) bevorzugt zur Sicherung gegen nasalen, oralen und/oder parenteralen, vorzugsweise intravenösen Missbrauch, die Komponente (b) bevorzugt gegen parenteralen, besonders bevorzugt intravenösen und/oder nasalen Missbrauch, die Komponente (c) bevorzugt gegen nasalen und/oder parenteralen, besonders bevorzugt intravenösen Missbrauch, die Komponente (d) vorzugsweise gegen parenteralen, besonders bevorzugt intravenösen, und/oder oralen und/oder nasalen Missbrauch, die Komponente (e) als visuelles Abschreckungsmittel gegen oralen oder parenteralen Missbrauch und die Komponente (f) gegen oralen oder nasalen Missbrauch. Durch die erfindungsgemäße Mitverwendung von wenigstens einer der vorstehend genannten Komponenten gelingt es, bei den durch das erfindungsgemäße Verfahren erhaltenen Darreichungsformen noch effektiver gegen Missbrauch vorzubeugen.

Beispielsweise kann die erfindungsgemäß erhaltene Darreichungsform auch zwei oder mehrere der Komponenten (a)-(f) in einer Kombination aufweisen, vorzugsweise (a), (b) und ggf. (c) und/oder (f) und/oder (e) bzw. (a), (b) und ggf. (d) und/oder (f) und/oder (e).

In einer weiteren Ausführungsform kann die erfindungsgemäße Darreichungsform sämtliche Komponenten (a)-(f) aufweisen.

Sofern die durch das erfindungsgemäße Verfahren erhaltene Darreichungsform eine Missbrauchs-verhindernde Komponente (a) umfasst, kommen als den Nasen-und/oder Rachenraum reizende Stoffe erfindungsgemäß sämtliche Stoffe in Betracht, die bei missbräuchlicher Applikation über den Nasen- und/oder Rachenraum eine Reaktion des Körpers hervorrufen, die entweder für den Missbraucher so unangenehm ist, dass der die Applikation nicht weiter fortsetzen will oder kann, so z. B. ein Brennen, oder auf physiologische Art und Weise einer Aufnahme des entsprechenden Wirkstoffes entgegenwirken, z. B. über eine vermehrte nasale Sekretbildung oder Niesen. Diese üblicherweise den Nasen- und/oder Rachenraum reizenden Stoffe können auch bei parenteraler, insbesondere intravenöser Applikation ein sehr unangenehmes Gefühl bis hin zu unerträglichen Schmerzen verursachen, so daß der Missbraucher die Einnahme nicht länger fortsetzen will oder kann.

Besonders geeignete, den Nasen- und/oder Rachenraum reizende Stoffe sind solche Stoffe, die ein Brennen, einen Juckreiz, einen Niesreiz, eine vermehrte Sekretbildung oder eine Kombination mindestens zweier dieser Reize verursachen. Entsprechende Stoffe und deren üblicherweise einzusetzenden Mengen sind dem Fachmann an sich bekannt oder können durch einfache Vorversuche ermittelt werden.

Der den Nasen- und/oder Rachenraum reizende Stoff der Komponente (a) basiert vorzugsweise auf einem oder mehreren Inhaltsstoffen oder einem oder mehreren Pflanzenteilen wenigstens einer Scharfstoffdroge.

Entsprechende Scharfstoffdrogen sind dem Fachmann an sich bekannt und werden beispielsweise in "Pharmazeutische Biologie - Drogen und ihre Inhaltsstoffe" von Prof. Dr. Hildebert Wagner, 2., bearbeitete Auflage, Gustav Fischer Verlag, Stuttgart-New York, 1982, Seiten 82 ff., beschrieben.

Vorzugsweise kann die erfindungsgemäß erhaltene Darreichungsform als Komponente (a) einer oder mehrere Inhaltsstoffe wenigstens einer Scharfstoffdroge ausgewählt aus der Gruppe umfassend Allii sativi Bulbus, Asari Rhizoma c. Herba, Calami Rhizoma, Capsici Fructus (Paprika), Capsici Fructus acer (Cayennepfeffer), Curcumae longae Rhizoma, Curcumae xanthorrhizae Rhizoma, Galangae Rhizoma, Myristicae Semen, Piperis nigri Fructus (Pfeffer), Sinapis albae (Erucae) Semen, Sinapis nigri Semen, Zedoariae Rhizoma und Zingiberis Rhizoma, besonders bevorzugt aus der Gruppe umfassend Capsici Fructus (Paprika), Capsici Fructus acer (Cayennepfeffer) und Piperis nigri Fructus (Pfeffer), hinzugefügt werden.

Bei den Inhaltsstoffen der Scharfstoffdrogen handelt es sich bevorzugt um o-Methoxy(Methyl)-phenol-Verbindungen, Säureamid-Verbindungen, Senföle oder Sulfidverbindungen oder um davon abgeleiteten Verbindungen.

Besonders bevorzugt ist wenigstens ein Inhaltsstoff der Scharfstoffdrogen ausgewählt aus der Gruppe bestehend aus Myristicin, Elemicin, Isoeugenol, α-Asaron, Safrol, Gingerolen, Xanthorrhizol, Capsaicinoiden, vorzugsweise Capsaicin, Capsaicin- Derivate, wie N-vanillyl -9E-octadecenamid, Dihydrocapsaicin, Nordihydrocapsaicin, Homocapsaicin, Norcapsaicin, und Nomorcapsaicin, Piperin, vorzugsweise trans-Piperin, Glucosinolaten, vorzugsweise auf Basis von nichtflüchtigen Senfölen, besonders bevorzugt auf Basis von p-Hydroxybenzylsenföl, Methylmercaptosenföl oder Methylsulfonylsenföl, und von diesen Inhaltsstoffen abgeleiteten Verbindungen.

Kommen ein oder mehrere Inhaltsstoffe entsprechender Scharfstoffdrogen zum Einsatz, beträgt deren Menge in einer durch das erfindungsgemäße Verfahren erhaltene Darreichungseinheit bevorzugt 0,001 bis 0,005 Gew.-%, bezogen auf das Gesamtgewicht der Darreichungseinheit.

Unter Darreichungseinheit wird eine separate bzw. separierbare Dosiseinheit, wie z. B. eine Tablette oder eine Kapsel, verstanden.

Vorzugsweise kann die das erfindungsgemäße Verfahren erhaltene Darreichungsform die Pflanzenteile der entsprechenden Scharfstoffdrogen in einer Menge von 0,01 bis 30 Gew.-%, besonders bevorzugt 0,1 bis 0,5 Gew.-%, jeweils bezogen auf das Gesamtgewicht der Darreichungseinheit, enthalten.

Eine weitere Möglichkeit, ei der erfindungsgemäß erhaltenen Darreichungsform gegen Missbrauch vorzubeugen, besteht darin, wenigstens ein viskositätserhöhendes Mittel als weitere Missbrauchs-verhindemde Komponente (b) der Darreichungsform zuzusetzen, das in einem mit Hilfe einer notwendigen Mindestmenge an einer wässrigen Flüssigkeit aus der Darreichungsform gewonnenen Extrakt ein Gel bildet, das kaum gefahrlos applizierbar ist und vorzugsweise beim Einbringen in eine weitere Menge einer wässrigen Flüssigkeit visuell unterscheidbar bleibt.

Visuelle Unterscheidbarkeit im Sinne der vorliegenden Erfindung bedeutet, dass das mit Hilfe einer notwendigen Mindestmenge an wässriger Flüssigkeit gebildete, wirkstoffhaltige Gel beim Einbringen vorzugsweise mit Hilfe einer Injektionsnadel, in eine weitere Menge wäßriger Flüssigkeit von 37°C im wesentlichen unlöslich und zusammenhängend bleibt und nicht auf einfache Weise so dispergiert werden kann, dass eine parenterale, insbesondere intravenöse, gefahrlose Applikation möglich ist. Vorzugsweise beträgt die Dauer der visuellen Unterscheidbarkeit wenigstens eine Minute, vorzugsweise mindestens 10 Minuten.

Die Viskositätserhöhung des Extrakts führt dazu, dass dessen Nadelgängigkeit bzw. Spritzbarkeit erschwert oder sogar unmöglich gemacht wird. Sofern das Gel visuell unterscheidbar bleibt, bedeutet dies, dass das erhaltene Gel beim Einbringen in eine weitere Menge wäßriger Flüssigkeit, z.B. durch Einspritzen in Blut, zunächst in Form eines weitgehend zusammenhängenden Fadens erhalten bleibt, der zwar durch mechanische Einwirkung in kleinere Bruchstücke zerteilt, nicht aber so dispergiert oder sogar gelöst werden kann, daß eine parenterale, insbesondere intravenöse, Applikation gefahrlos möglich ist. In Kombination mit mindestens einer ggf. vorhandenen Komponente (a) bzw. c bis (e) führt dies zusätzlich zu unangenehmen Brennen, Erbrechen, schlechtem Geschmack und/oder zur visuellen Abschreckung.

Eine intravenöse Applikation eines entsprechenden Gels würde daher mit großer Wahrscheinlichkeit zu schweren gesundheitlichen Beeinträchtigungen des Missbrauchers führen.

Zur Überprüfung, ob ein viskositätserhöhendes Mittel als Komponente (b) zur Anwendung in der erfindungsgemäß erhaltenen Darreichungsform geeignet ist, wird der Wirkstoff mit dem viskositätserhöhenden Mittel gemischt und in 10 ml Wasser bei einer Temperatur von 25°C suspendiert. Bildet sich hierbei ein Gel, welches den obenstehend genannten Bedingungen genügt, eignet sich das entsprechende viskositätserhöhende Mittel zur Missbrauchs-Vorbeugung bzw. - Verhinderung bei den erfindungsgemäß erhaltenen Darreichungsformen.

Sofern der erfindungsgemäß erhaltenen Darreichungsform die Komponente (b) hinzugefügt wird, kommen vorzugsweise eine oder mehrere viskositätserhöhende Mittel zum Einsatz, die ausgewählt sind aus der Gruppe umfassend mikrokristalliner Cellulose mit 11 Gew.-% Carboxymethylcellulose-Natrium (Avicel^{®} RC 591), Carboxymethylcellulose-Natrium (Blanose^{®}, CMC-Na C300P^{®}, Frimulsion BLC-5^{®}, Tylose C300 P^{®}), Polyacrylsäure (Carbopol^{®} 980 NF, Carbopol^{®} 981), Johannisbrotkernmehl (Cesagum^{®} LA-200, Cesagum^{®} LID/150, Cesagum^{®} LN-1), Pektine wie Citrus-Pectin (Cesapectin^{®} HM Medium Rapid Set), Apfelpektin, Pektin aus Zitronenschale, Wachsmaisstärke (C*Gel 04201^{®}), Natriumalginat (Frimulsion ALG (E401)^{®}), Guarkernmehl (Frimulsion BM^{®}, Polygum 26/1-75^{®}), lota-Carrageen (Frimulsion D021^{®}), Karaya Gummi, Gellangummi (Kelcogel F^{®}, Kelcogel LT100^{®}), Galaktomannan (Meyprogat 150 ^{®}), Tarakemmehl (Polygum 43/1^{®}), Propylenglykoalginat (Protanal-Ester SD-LB^{®}),,
Natrium-Hyaluronat, Tragant, Taragummi (Vidogum SP 200^{®}), fermentiertes Polysaccharid- Welan Gum (K1A96), Xanthan-Gummi (Xantural 180^{®}). Xanthane sind besonders bevorzugt. Die in Klammern angegebenen Bezeichnungen sind die Handelsnamen, unter denen die jeweiligen Materialien am Markt geführt sind. Im allgemeinen ist eine Menge von 0,1 bis 5 Gew.% der/des genannten viskositätserhöhenden Mittels ausreichend, um die vorstehend genannten Bedingungen zu erfüllen.

Die viskositätserhöhenden Mittel der Komponente (b), sofern vorgesehen, liegen in der erfindungsgemäß erhaltenen Darreichungsform bevorzugt in Mengen von mindestens 5 mg pro Darreichungseinheit, d.h. pro Dosiereinheit vor.

In einer besonders bevorzugten Ausführungsform der vorliegenden Erfindung kommen als Komponente (b) solche viskositätserhöhenden Mittel zum Einsatz, die bei der Extraktion aus der Darreichungsform mit der notwendigen Mindestmenge an wäßriger Flüssigkeit ein Gel bilden, das Luftblasen einschließt. Die so erhaltenen Gele zeichnen sich durch ein trübes Erscheinungsbild aus, durch das der potentielle Missbraucher zusätzlich optisch gewarnt und von dessen parenteraler Applikation abgehalten wird.

Die Komponente (C) kann auch gegebenenfalls als zusätzliches viskositätserhöhendes Mittel dienen, das mit Hilfe einer notwendigen Mindestmenge an einer wässrigen Flüssigkeit ein Gel bildet.

Es ist auch möglich, die viskositätserhöhenden Mittel und die übrigen Bestandteile in räumlich voneinander getrennter Anordnung in der erfindungsgemäß erhaltenen Darreichungsform zu formulieren.

Des weiteren kann die erfindungsgemäß erhaltene Darreichungsform zur Vorbeugung und Sicherung gegen Missbrauch die Komponente (c) aufweisen, nämlich einen oder mehrere Antagonisten für den Wirkstoff bzw. die Wirkstoffe mit Missbrauchspotential, wobei die Antagonistenmenge vorzugsweise räumlich getrennt von den übrigen Bestandteilen der erfindungsgemäß erhaltenen Darreichungsform vorliegen und keine Wirkung bei bestimmungsgemäßer Verwendung entfalten.

Geeignete Antagonisten zur Verhinderung des Missbrauchs der Wirkstoffe sind dem Fachmann bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäß erhaltenen Darreichungsform vorliegen.

Sofern der in der Darreichungsform vorliegende Wirkstoff ein Opioid ist, kommt als Antagonist bevorzugt ein Antagonist ausgewählt aus der Gruppe umfassend Naloxon, Naltrexon, Nalmefen, Nalid, Nalmexon, Nalorphin oder Naluphin, jeweils ggf. in Form einer entsprechenden physiologisch verträglichen Verbindung, insbesondere in Form einer Base, eines Salzes oder Solvates, zum Einsatz. Vorzugsweise werden die entsprechenden Antagonisten, sofern eine Ausrüstung mit der Komponente (c) vorgesehen ist, in einer Menge von mindestens 1 mg, besonders bevorzugt in einer Menge von 3 bis 100 mg, ganz besonders bevorzugt in einer Menge von 5 bis 50 mg auf pro Darreichungsform, d.h. pro Dosiereinheit eingesetzt.

Weist die erfindungsgemäß erhaltene Darreichungsform als Wirkstoff ein Stimulanz auf, ist der Antagonist bevorzugt ein Neuroleptikum, vorzugsweise wenigstens eine Verbindung ausgewählt aus der Gruppe umfassend Haloperidol, Promethacin, Fluophenozin, Perphenazin, Levomepromazin, Thioridazin, Perazin, Chlorpromazin, Chlorprothixen, Zuclopentixol, Flupentixol, Prothipendyl, Zotepin, Benperidol, Piparmeron, Melperol und Bromperidol.

Vorzugsweise weist die erfindungsgemäß erhaltene Darreichungsform diese Antagonisten in einer üblichen, dem Fachmann bekannten therapeutischen Dosierung, besonders bevorzugt in einer gegenüber der üblichen Dosierung verdoppelten bis verdreifachten Menge pro Dosiereinheit auf.

Sofern die Kombination zur Vorbeugung und Sicherung der erfindungsgemäß erhaltenen Darreichungsform gegen Missbrauch die Komponente (d) umfaßt, kann sie wenigstens ein Emetikum aufweisen, das vorzugsweise in einer räumlich getrennten Anordnung von den übrigen Komponenten der erfindungsgemäß erhaltenen Darreichungsform vorliegen und bei bestimmungsgemäßer Anwendung keine Wirkung im Körper entfalten sollte.

Geeignete Emetika zur Verhinderung des Missbrauchs eines Wirkstoffs sind dem Fachmann bekannt und können als solche oder in Form entsprechender Derivate, insbesondere Ester oder Ether, oder jeweils in Form entsprechender physiologisch verträglicher Verbindungen, insbesondere in Form ihrer Salze oder Solvate in der erfindungsgemäß erhaltenen Darreichungsform vorliegen.

In der erfindungsgemäß erhaltenen Darreichungsform kann bevorzugt ein Emetikum auf Basis eines oder mehrerer Inhaltsstoffe von Radix Ipecacuanhae (Brechwurzel), vorzugsweise auf Basis des Inhaltsstoffes Emetin, in Betracht, wie sie z.B. in "Pharmazeutische Biologie - Drogen und ihre Inhaltsstoffe" von Prof. Dr. Hildebert Wagner, 2., bearbeitete Auflage, Gustav Fischer Verlag, Stuttgart, New York 1982 beschrieben werden.

Vorzugsweise kann die erfindungsgemäß erhaltene Darreichungsform als Komponente (d) das Emetikum Emetin aufweisen, bevorzugt in einer Menge von wenigstens 3 mg, besonders bevorzugt wenigstens 10 mg und ganz besonders bevorzugt in einer Menge von wenigstens 20 mg pro Darreichungsform, d. h. Dosiereinheit.

Ebenfalls bevorzugt kann als Emetikum Apomorphin als zusätzliche Missbrauchssicherung zum Einsatz kommen, vorzugsweise in einer Menge von vorzugsweise wenigstens 3 mg, besonders bevorzugt wenigstens 5 mg und ganz besonders bevorzugt wenigstens 7 mg pro Dosiereinheit.

Sofern die erfindungsgemäß erhaltene Darreichungsform die Komponente (e) als weiteren missbrauchsverhindemden Hilfsstoff enthält, so wird durch den Einsatz eines solchen Farbstoffes, insbesondere bei dem Versuch, den Wirkstoff für eine parenterale, vorzugsweise intravenöse Applikation, zu extrahieren, eine intensive Farbgebung einer entsprechenden wässrigen Lösung hervorgerufen, die zur Abschreckung beim potentiellen Missbraucher führen kann. Auch ein oraler Missbrauch, der üblicherweise über eine wässrige Extraktion des Wirkstoffes eingeleitet wird, kann durch diese Farbgebung verhindert werden. Geeignete Farbstoffe sowie die für die notwendige Abschreckungswirkung erforderlichen Mengen sind der WO 03/015531 zu entnehmen.

Sofern die erfindungsgemäß erhaltene Darreichungsform als weiteren Missbrauchsverhindernden Hilfsstoff die Komponente (f) enthält, so wird durch diesen Zusatz von wenigstens einem Bitterstoff durch die damit eintretende Geschmacksverschlechterung der Darreichungsform der orale und/oder nasale Missbrauch zusätzlich verhindert.

Geeignete Bitterstoffe sowie die für den Einsatz wirksamen Mengen sind der US-2003/0064099 A1 zu entnehmen. Vorzugsweise eignen sich als Bitterstoffe Aromaöle, vorzugsweise Pfefferminzöl, Eukalyptusöl, Bittermandelöl, Menthol, Fruchtaromastoffe, vorzugsweise Aromastoffe von Zitronen, Orangen, Limonen, Grapefruit oder Mischungen davon, und/oder Denatonium-Benzoat (Bitrex ®). Besonders bevorzugt wird Denatonium-Benzoat eingesetzt.

Die erfindungsgemäß erhaltene feste Darreichungsform eignet sich zur oralen, vaginalen oder rektalen, vorzugsweise zur oralen Einnahme von Mensch und Tier.

Die oral applizierbare, erfindungsgemäß erhaltene Darreichungsform kann wie erwähnt in multipartikulärer Form, bevorzugt in Form von Granulaten, vorzugsweise in zylindrischer Form, Spheroiden, Perlen oder Pellets, ggf. in Kapseln abgefüllt oder zu Tabletten verpresst, vorliegen. Vorzugsweise weisen die multipartikulären Formen eine Mindestgröße von 0,1 mm bis 10 cm, besonders bevorzugt eine Größe von 0,1 bis 3,0 mm, besonders bevorzugt eine Größe von 0,5 bis 2 mm auf.

In einer besonders bevorzugten Ausführungsform liegt die erfindungsgemäß erhaltene Darreichungsform in Form einer Tablette, einer Kapsel oder in Form eines oralen osmotischen therapeutischen Systems (OROS) vor, vorzugsweise wenn mindestens noch eine weitere missbrauchsverhindernde Komponente (a) - (f) vorhanden ist.

Sofern die Komponenten (c) und/oder (d) und/oder (f) in der erfindungsgemäß hergestellte Darreichungsform vorhanden sind, ist darauf zu achten, dass sie so formuliert oder so gering dosiert sind, dass sie bei bestimmungsgemäßer Applikation der Darreichungsform praktisch keine den Patienten oder die Wirksamkeit des Wirkstoffs beeinträchtigende Wirkung entfalten können.

Sofern die erfindungsgemäß hergestellte Darreichungsform die Komponente (d) und/oder (f) enthält, ist die Dosierung so zu wählen, dass bei bestimmungsgemäßer oraler Applikation keine negative Wirkung hervorgerufen wird. Wird jedoch die vorgesehene Dosierung der Darreichungsform versehentlich, insbesondere durch Kinder, oder beim Missbrauch überschritten, wird Übelkeit bzw. Brechreiz bzw. schlechter Geschmack hervorgerufen. Die jeweilige Menge der Komponente (d) und/oder (f), die vom Patienten bei bestimmungsgemäßer oraler Applikation noch toleriert wird, kann vom Fachmann durch einfache Vorversuche ermittelt werden.

Sofern aber unabhängig von der praktisch nicht möglichen Pulverisierbarkeit der erfindungsgemäß hergestellten Darreichungsform zur Sicherung der Darreichungsform enthaltend die Komponenten (c) und/oder (d) und/oder (f) vorgesehen ist, sollten diese Komponenten bevorzugt in einer so hohen Dosierung zum Einsatz kommen, dass sie bei einer missbräuchlichen Applikation der Darreichungsform eine intensive negative Wirkung beim Missbraucher hervorrufen. Dies gelingt vorzugsweise durch eine räumliche Trennung zumindest des Wirkstoffes bzw. der Wirkstoffe von den Komponenten (c) und/oder (d) und/oder (f), wobei bevorzugt der Wirkstoff bzw. die Wirkstoffe in wenigstens einer Untereinheit (X) und die Komponenten (c) und/oder (d) und/oder (f) in wenigstens einer Untereinheit (Y) vorliegen, und wobei die Komponenten (c), (d) und (f) bei bestimmungsgemäßer Applikation der Darreichungsform bei Einnahme und/oder im Körper nicht ihre Wirkung entfalten und die übrigen Formulierungskomponenten insbesondere die Komponente (C) identisch sind.

Sofern die erfindungsgemäße Darreichungsform wenigstens 2 der Komponenten (c) und (d) bzw. (f) aufweist, können diese jeweils in derselben oder in verschiedenen Untereinheiten (Y) vorliegen. Vorzugsweise liegen, sofern vorhanden, alle Komponenten (c) und (d) und (f) in ein- und derselben Untereinheit (Y) vor.

Untereinheiten im Sinne der vorliegenden Erfindung sind feste Formulierungen, die jeweils neben üblichen, dem Fachmann bekannten Hilfsstoffen den (die) Wirkstoff(e), vorzugsweise auch mindestens ein Polymer (C) und gegebenenfalls wenigstens eine der gegebenenfalls vorhandenen Komponenten (a) und/oder (b) und/oder (e) bzw. vorzugsweise jeweils wenigstens ein Polymer (C) und den (die) Antagonist(en) und/oder das Emetikum (die Emetika) und/oder die Komponente (e) und/oder die Komponente (f) und gegebenenfalls wenigstens eine der gegebenenfalls vorhandenen Komponenten (a) und/oder (b) enthalten. Dabei ist darauf zu achten, dass vorzugsweise jede der Untereinheiten nach dem vorstehend angegebenen, erfindungsgemäßen Verfahren formuliert werden, sofern die mechanische Fertigkeit gewünscht bzw. erforderlich ist.

Ein wesentlicher Vorteil der getrennten Formulierung der Wirkstoffe von den Komponenten (c) bzw. (d) bzw. (f) in Untereinheiten (X) und (Y) der erfindungsgemäß hergestellten Darreichungsform besteht darin, dass bei ihrer bestimmungsgemäßen Applikation die Komponenten (c) und/oder (d) und/oder (f) bei Einnahme und/oder im Körper praktisch nicht freigesetzt werden oder nur in so geringen Mengen freigesetzt werden, dass sie keine den Patienten oder den Therapieerfolg beeinträchtigende Wirkung entfalten oder bei der Passage durch den Körper des Patienten nur an solchen Freisetzungsorten abgegeben werden, an denen eine für ihre Wirksamkeit ausreichende Resorption nicht gegeben ist. Vorzugsweise werden die Komponenten (c) und/oder (d) und/oder (f) bei bestimmungsgemäßer Applikation der Darreichungsform im Körper des Patienten praktisch nicht freigesetzt oder vom Patienten nicht wahrgenommen.

Der Fachmann versteht, dass diese vorstehend genannten Bedingungen in Abhängigkeit von den jeweils eingesetzten Komponenten (c), (d) und/oder (f) sowie der Formulierung der Untereinheiten bzw. der Darreichungsform variieren können. Die für die jeweilige Darreichungsform optimale Formulierung kann durch einfache Vorversuche ermittelt werden. Entscheidend ist, wenn erforderlich zur Missbrauchsverhinderung, dass die jeweiligen Untereinheiten das Polymer (C) und ggf. (D) enthalten und in der angegebenen Weise formuliert und erfindungsgemäß hergestellt wurden.

Sollte es den Missbrauchern wider Erwarten gelingen, eine solche erfindungsgemäß erhaltene Darreichungsform, welche die Komponenten (c) und/oder (e) und/oder (d) und/oder (f) in Untereinheiten (Y) aufweist, zum Zwecke der missbräuchlichen Einnahme des Wirkstoffes zu zerkleinern und ein Pulver zu erhalten, das mit einem geeigneten Extraktionsmittel extrahiert wird, wird neben dem Wirkstoff auch die jeweilige Komponente (c) und/oder (e) und/oder (f) und/oder (d) in einer Form erhalten, in der sie von dem Wirkstoff nicht auf einfache Weise zu separieren ist, so dass sie bei der Applikation der manipulierten Darreichungsform, insbesondere bei oraler und/oder parenteraler Verabreichung, ihre Wirkung bei Einnahme und/oder im Körper entfaltet und zusätzlich eine der Komponente (c) und/oder (d) und/oder (f) entsprechende negative Wirkung beim Missbraucher hervorruft oder ein Versuch, den Wirkstoff zu extrahieren durch die Farbgebung abschreckt und so den Missbrauch der Darreichungsform verhindert.

Die erfindungsgemäße Formulierung einer Darreichungsform, in der eine räumliche Trennung des Wirkstoffes bzw. der Wirkstoffe von den Komponenten (c), (d) und/oder (e), vorzugsweise durch Formulierung in verschiedenen Untereinheiten erfolgt ist, kann in vielfältiger Art und Weise erfolgen, wobei die entsprechenden Untereinheiten einer solchen Darreichungsform jeweils in beliebiger räumlicher Anordnung zueinander vorliegen können, sofern die vorstehend genannten Bedingungen für die Freisetzung der Komponenten (c) und/oder (d) erfüllt sind.

Der Fachmann versteht, dass die ggf. auch vorliegenden Komponente(n) (a) und/oder (b) bevorzugt sowohl in den jeweiligen Untereinheiten (X) und (Y) als auch in Form von eigenständigen, den Untereinheiten (X) und (Y) entsprechenden Untereinheiten in der erfindungsgemäß hergestellten Darreichungsform formuliert werden können, so lange die Sicherung der Darreichungsform gegen den Missbrauch wie auch die Wirkstofffreisetzung bei bestimmungsgemäßer Applikation durch die Art der Formulierung nicht beeinträchtig werden und das Polymer (C) vorzugsweise mit formuliert und die Formulierung vorzugsweise gemäß dem erfindungsgemäßen Verfahren durchgeführt wird.

In einer bevorzugten Ausführungsform der erfindungsgemäß hergestellten Darreichungsform liegen die Untereinheiten (X) und (Y) in multipartikulärer Form vor, wobei Granulate, Sphäroide, Perlen oder Pellets bevorzugt sind und sowohl für die Untereinheit (X) als auch (Y) dieselbe Form, d.h. Gestaltung gewählt wird, damit keine Separierung der Untereinheiten (X) von (Y) durch mechanische Auslese möglich ist. Die multipartikulären Formen weisen bevorzugt eine Größe im Bereich von 0,1 bis 3 mm, vorzugsweise 0,5 bis 2 mm auf.

Die Untereinheiten (X) und (Y) in multpartikulärer Form können auch bevorzugt in eine Kapsel abgefüllt oder zu einer Tablette verpresst werden, wobei die jeweiligen Endformulierungen dergestalt erfolgen, dass die Untereinheiten (X) und (Y) auch in der resultierenden Darreichungsform erhalten bleiben.

Die jeweiligen multipartikulären Untereinheiten (X) bzw. (Y) mit identischer Formgebung sollten auch nicht visuell voneinander unterscheidbar sein, damit sie vom Missbraucher nicht durch einfaches Sortieren voneinander separiert werden können. Dies kann beispielsweise durch das Aufbringen identischer Überzüge gewährleistet werden, die neben dieser Egalisierungsfunktion auch weitere Funktionen übernehmen können, wie z.B. die Retardierung eines oder mehrerer Wirkstoffe oder eine magensaftresistente Ausrüstung der jeweiligen Untereinheiten.

In einer weiteren bevorzugten Ausführungsform der vorliegenden Erfindung sind die Untereinheiten (X) und (Y) jeweils schichtförmig zueinander angeordnet.

Bevorzugt sind hierfür die schichtförmigen Untereinheiten (X) und (Y) in der erfindungsgemäß hergestellten Darreichungsform vertikal oder horizontal zueinander angeordnet, wobei jeweils auch eine oder mehrere schichtförmige Untereinheiten (X) und eine oder mehrere schichtförmige Untereinheiten (Y) in der Darreichungsform vorliegen können, so dass neben den bevorzugten Schichtenfolgen (X)-(Y) bzw. (X)-(Y)-(X) beliebige andere Schichtenfolgen in Betracht kommen, ggf. in Kombination mit Schichten enthaltend die Komponenten (a) und/oder (b).

Ebenfalls bevorzugt ist eine erfindungsgemäß hergestellte Darreichungsform, in der die Untereinheit (Y) einen Kern bildet, der von der Untereinheit (X) vollständig umhüllt wird, wobei zwischen diesen Schichten eine Trennschicht (Z) vorhanden sein kann. Ein entsprechender Aufbau eignet sich bevorzugt auch für die vorstehend genannten multipartikulären Formen, wobei dann beide Untereinheiten (X) und (Y) sowie eine ggf. vorhandene Trennschicht (Z), die vorzugsweise der erfindungsgemäßen Härteanforderung genügen sollte, in ein- und derselben multipartikulären Form nach dem erfindungsgemäßen Verfahren formuliert werden.

In einer weiteren bevorzugten Ausführungsform der erfindungsgemäß hergestellten Darreichungsform bildet die Untereinheit (X) einen Kern, der von der Untereinheit (Y) umhüllt wird, wobei letztere wenigstens einen Kanal aufweist, der von dem Kern an die Oberfläche der Darreichungsform führt.

Zwischen einer Schicht der Untereinheit (X) und einer Schicht der Untereinheit (Y) kann die erfindungsgemäß hergestellten Darreichungsform jeweils eine oder mehrere, vorzugsweise eine, ggf. quellbare Trennschicht (Z) zur räumlichen Trennung der Untereinheit (X) von (Y) aufweisen.

Sofern die erfindungsgemäß hergestellte Darreichungsform die schichtförmigen Untereinheiten (X) und (Y) sowie eine ggf. vorhandene Trennschicht (Z) in einer zumindest teilweise vertikalen oder horizontalen Anordnung aufweist, liegt sie bevorzugt in Form einer Tablette, eines Coextrudats oder Laminats vor, das nach dem erfindungsgemäßen Verfahren hergestellt wurde.

Hierbei kann in einer besonders bevorzugten Ausführungsform die freie Oberfläche der Untereinheit (Y) vollständig und ggf. zumindest ein Teil der freien Oberfläche der Untereinheit(en) (X) und ggf. zumindest ein Teil der freien Oberfläche der ggf. vorhandenen Trennschicht(en) (Z) mit wenigstens einer die Freisetzung der Komponente (c) und/oder (e) und/oder (d) und/oder (f) verhindernden Barriereschicht (Z') überzogen sein. Auch die Barriereschicht (Z') sollte vorzugsweise die erfindungsgemäßen Härtevoraussetzungen erfüllen.

Ebenfalls besonders bevorzugt ist eine Ausführungsform der erfindungsgemäß hergestellten Darreichungsform, die eine vertikale oder horizontale Anordnung der Schichten der Untereinheiten (X) und (Y) und wenigstens eine dazwischen angeordnete Push-Schicht (p) sowie ggf. eine Trennschicht (Z) aufweist, in der sämtliche freie Oberflächen des aus den Untereinheiten (X) und (Y), der Push-Schicht und der ggf. vorhandenen Trennschicht (Z) bestehenden Schichtaufbaus mit einem semipermeablen Überzug (E) ausgerüstet sind, der für ein Freisetzungsmedium, d.h. üblicherweise eine physiologische Flüssigkeit, durchlässig, für den Wirkstoff und für die Komponente (c) und/oder (d) und/oder (f) im wesentlichen undurchlässig ist, und wobei dieser Überzug (E) im Bereich der Untereinheit (X) wenigstens eine Öffnung zur Freisetzung des Wirkstoffes aufweist.

Eine entsprechende Darreichungsform ist dem Fachmann beispielsweise unter der Bezeichnung orales osmotisches therapeutisches System (OROS), ebenso wie geeignete Materialien und Verfahren zu dessen Herstellung, u.a. aus US 4,612,008, US 4,765,989 und US 4,783,337 bekannt.

In einer weiteren bevorzugten Ausführungsform hat die Untereinheit (X) der erfindungsgemäß hergestellten Darreichungsform die Form einer Tablette, deren Steg und ggf. eine der beiden Grundflächen mit einer die Komponente (c) und/oder (d) und/oder (f) enthaltenden Barriereschicht (Z') bedeckt ist.

Der Fachmann versteht, dass die bei der erfindungsgemäßen Herstellung der jeweiligen Darreichungsformen jeweils zum Einsatz kommenden Hilfsstoffe der Untereinheit(en) (X) bzw. (Y) sowie ggf. der vorhandenen Trennschicht(en) (Z) und/oder der Barriereschicht(en) (Z') in Abhängigkeit von deren Anordnung in der Darreichungsform, der Applikationsart sowie in Abhängigkeit von dem jeweiligen Wirkstoff der ggf. vorhandenen Komponenten (a) und/oder (b) und/oder (e) und der Komponente (c) und/oder (d) und/oder (f) variieren. Die Materialien, die über die jeweils erforderlichen Eigenschaften verfügen sind, dem Fachmann an sich bekannt.

Sofern die Freisetzung der Komponente (c) und/oder (d) und/oder (f) aus der Untereinheit (Y) der erfindungsgemäß hergestellten Darreichungsform mit Hilfe einer Umhüllung, vorzugsweise einer Barriereschicht, verhindert wird, kann die Untereinheit aus üblichen, dem Fachmann bekannten Materialien bestehen, vorzugsweise wenigstens ein Polymer(C) enthalten und vorzugsweise erfindungsgemäß hergestellt sein.

Ist eine entsprechende Barriereschicht (Z') zur Verhinderung der Freisetzung der Komponente (c) und/oder (d) und/oder (f) nicht vorgesehen, sind die Materialien der Untereinheiten so zu wählen, dass eine Freisetzung der jeweiligen Komponente (c) und/oder (d) aus der Untereinheit (Y) praktisch ausgeschlossen ist.

Bevorzugt können hierzu die nachstehend aufgeführten Materialien zum Einsatz kommen, die auch für den Aufbau der Barriereschicht geeignet sind und sollten vorzugsweise wenigstens ein Polymer (C) zur Erfüllung der Härtebedingungen enthalten.

Bevorzugte Materialien sind solche, die ausgewählt sind aus der Gruppe bestehend aus Alkylcellulosen, Hydroxyalkylcellulosen, Glucanen, Skleroglucanen, Mannanen, Xanthanen, Copolymeren aus Poly[bis(p-carboxyphenoxy)propan und Sebacinsäure vorzugsweise in einem Molverhältnis von 20:80 (unter der Bezeichnung Polifeprosan 20^{®} am Markt geführt), Carboxymethylcellulosen, Celluloseethern, Celluloseestern, Nitrocellulosen, Polymeren auf Basis von (Meth)acrylsäure sowie deren Estern, Polyamiden, Polycarbonaten, Polyalkylenen, Polyalkylenglykolen, Polyalkylenoxiden, Polyalkylenterephtalate, Polyvinylalkohole, Polyvinylether, Polyvinylester, halogenierte Polyvinyle, Polyglykolide, Polysiloxane sowie Polyurethane und deren Copolymeren.

Besonders geeignete Materialien können ausgewählt werden aus der Gruppe bestehend aus Methylcellulose, Ethylcellulose, Hydroxypropylcellulose, Hydroxypropylmethylcellulose, Hydroxybutylmethylcellulose, Celluloseacetat, Cellulosepropionat (von niederem, mittlerem oder erhöhtem Molekulargewicht), Celluloseacetatpropionat, Celluloseacetatbutyrat, Celluloseacetatphtalat, Carboxymethylcellulose, Cellulosetriacetat, Natrium-Cellulosesulfat, Polymethylmethacrylat, Polyethylmethacrylat, Polybutylmethacrylat, Polyisobutylmethacrylat, Polyhexylmethacrylat, Polyisodecylmethacrylat, Polylaurylmethacrylat, Polyphenylmethacrylat, Polymethylacrylat, Polyisopropylacrylat, Polyisobutylacrylat, Polyoctatdecylacrylat, Polyethylen, Polyethylen niederer Dichte, Polyethylen hoher Dichte, Polypropylen, Polyethylenglykol, Polyethylenoxid, Polyethylenterephtalat, Polyvinylalkohol, Polyvinylisobutylether, Polyvinylacetat und Polyvinylchlorid.

Besonders geeignete Copolymere können ausgewählt werden aus der Gruppe bestehend aus Copolymeren aus Butylmethacrylat und Isobutylmethacrylat, Copolymeren aus Methylvinylether und Maleinsäure mit erhöhtem Molekulargewicht, Copolymeren aus Methylvinylether und Maleinsäuremonoethylester, Copolymeren aus Methylvinylether und Maleinsäureanhydrid sowie Copolymeren aus Vinylalkohol und Vinylacetat.

Weitere, zur Formulierung der Barriereschicht besonders geeignete Materialien sind Stärke gefülltes Polycaprolacton (WO98/20073), aliphatische Polyesteramide (DE 19 753 534 A1, DE 19 800 698 A1, EP 0 820 698 A1), aliphatische und aromatische Polyesterurethane (DE 19822979), Polyhydroxyalkanoate, insbesondere Polyhydroxybutyrate, Polyhydroxyvaleriate), Casein (DE 4 309 528), Polylactide und Copolylactide (EP 0 980 894 A1).

Ggf. können die vorstehend genannten Materialien mit weiteren üblichen, dem Fachmann bekannten Hilfsstoffen, vorzugsweise ausgewählt aus der Gruppe bestehend aus Glycerylmonostearat, halbsynthetischen Triglyceridderivaten, halbsynthetischen Glyceriden, hydriertem Rizinusöl, Glycerylpalmitostearat, Glycerylbehenat, Polyvinylpyrrolidon, Gelatine, Magnesiumstearat, Stearinsäure, Natriumstearat, Talkum, Natriumbenzoat, Borsäure und kolloidalem Silica, Fettsäuren, substituierten Triglyceriden, Glyceriden, Polyoxyalkylenglykolen und deren Derivate abgemischt werden.

Sofern die erfindungsgemäß hergestellte Darreichungsform eine Trennschicht (Z') aufweist, kann diese, ebenso wie die nicht umhüllte Untereinheit (Y) vorzugsweise aus den vorstehend, für die Barriereschicht beschriebenen Materialien bestehen. Der Fachmann versteht, daß auch über die Dicke der Trennschicht die Freisetzung des Wirkstoffes bzw. der Komponente (c) und/oder (d) aus der jeweiligen Untereinheit gesteuert werden kann.

Die erfindungsgemäß hergestellte Darreichungsform kann einen oder mehrere Wirkstoffe zumindest teilweise in retardierter Form aufweisen, wobei die Retardierung mit Hilfe von üblichen, dem Fachmann bekannten Materialien und Verfahren erzielt werden kann, beispielsweise durch Einbetten des Wirkstoffes in eine retardierende Matrix oder durch das Aufbringen eines oder mehrerer retardierender Überzüge. Die Wirkstoffabgabe muss aber so gesteuert sein, dass die vorstehend genannten Bedingungen jeweils erfüllt sind, z.B. das bei bestimmungsgemäßer Applikation der Darreichungsform der Wirkstoff bzw. die Wirkstoffe praktisch komplett freigesetzt wird, bevor die ggf. vorhandenen Komponente (c) und/oder (d) eine beeinträchtigende Wirkung entfalten können. Durch die Retardierung kann die Wirkstoff-Freiseetzung so gesteuert werden, dass eine zweimal oder einmal tägliche Verabreichung der Darreichungsform reicht, was insbesondere bei opioid-haltigen Wirkstoffen zur Schmerzbekämpfung vorteilhaft ist.

Die kontrollierte Freisetzung aus der erfindungsgemäß hergestellten Darreichungsform wird vorzugsweise durch Einbettung des Wirkstoffes in eine Matrix erzielt. Die als Matrixmaterialien dienenden Hilfsstoffe kontrollieren die Wirkstofffreisetzung. Matrixmaterialien können beispielweise hydrophile, gelbildende Materialien sein, woraus die Wirkstofffreisetzung hauptsächlich durch Diffusion erfolgt, oder hydrophobe Materialien sein, woraus die Wirkstofffreisetzung hauptsächlich durch Diffusion aus den Poren in der Matrix erfolgt.

Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophoben Polymeren, Wachsen, Fetten, langkettigen Fettsäuren, Fettalkoholen oder entsprechenden Estern oder Ethern oder deren Gemische. Besonders bevorzugt werden als hydrophobe Materialien Mono- oder Digliceride von C12-C30-Fettsäuren und/oder C12-C30-Fettalkohole und/oder Wachse oder deren Gemische eingesetzt.

Es ist auch möglich, Mischungen der vorstehend genannten hydrophilen und hydrophoben Materialien als Matrixmaterialien einzusetzen.

Des weiteren können auch die Komponenten (C) und ggf. vorhandene Komponente (D) als ggf. zusätzliche Matrixmaterialien dienen.

Sofern die erfindungsgemäß hergestellte Darreichungsform zur oralen Applikation vorgesehen ist, kann sie bevorzugt auch einen magensaftresistenten Überzug aufweisen, der sich in Abhängigkeit vom pH-Wert der Freisetzungsumgebung auflöst. Durch diesen Überzug kann erreicht werden, dass die erfindungsgemäß hergestellte Darreichungsform den Magentrakt unaufgelöst passiert und der Wirkstoff erst im Darmtrakt zur Freisetzung gelangt. Vorzugsweise löst sich der magensaftresistente Überzug bei einem pH-Wert zwischen 5 und 7,5 auf. Mit retardierenden und/oder geschmackskaschierenden Überzügen können die erfindungsgemäß hergestellten Darreichungsformen ebenfalls versehen werden.

Entsprechende Materialien und Verfahren zur Retardierung von Wirkstoffen sowie zum Aufbringen magensaftresistenter und/oder retardierenden Überzüge sind dem Fachmann beispielsweise aus "Coated Pharmaceutical Dosage Forms - Fundamentals, Manufacturing Techniques, Biopharmaceutical Aspects, Test Methods and Raw Materials" von Kurt H. Bauer, K. Lehmann, Hermann P. Osterwald, Rothgang, Gerhart, 1. Auflage, 1998, Medpharm Scientific Publishers bekannt.

### Bezugszeichenliste

- 1: Welle
- 2: Flussrichtung (Extrusionsrichtung) des Produktes (Mischung)
- 3: Zentralspindel mit Spindelkopf 32
- 31: Flanke der Zentralspindel
- 4: Planet-Spindel
- 41: Flanke der Planet-Spindel
- 42: Drehrichtung der Planet-Spindel um die eigene Achse
- 43: Drehrichtung der Planet-Spindel um die Zentralspindel
- 5: Einzugsschnecke
- 6: Gehäuse (Mantel)
- 61: Flanke des Gehäuses
- 7: Einfüllvorrichtung
- 8: Düsenbereich
- 9: Extrusionsbereich

### Methode zur Bestimmung der Bruchfestigkeit

Zur Überprüfung, ob ein Material als Komponente (C) oder (D) eingesetzt werden kann, wird das Material zu einer Tablette mit einem Durchmesser von 10 mm und einer Höhe von 5mm mit einer Kraft von 150 N, bei einer Temperatur entsprechend mindestens dem Erweichungspunkt des Polymeren und bestimmt mit Hilfe eines DSC-Diagramms des Materials verpresst. Mit so hergestellten Tabletten wird gemäß der Methode zur Bestimmung der Bruchfestigkeit von Tabletten, veröffentlicht im Europäischen Arzneibuch 1997, Seite 143, 144, Methode Nr. 2.9.8. unter Einsatz der nachstehend aufgeführten Apparatur die Bruchfestigkeit bestimmt. Als Apparatur für die Messung wird eine Zwick Materialprüfmaschine "Zwick Z 2.5", Materialprüfmaschine Fmax 2.5 kN mit einem Traversenweg von max. 1150 mm, der durch einen Aufbau mit Hilfe einer Säule und einer Spindel einzustellen ist, einen freien Arbeitsraum nach hinten von 100 mm und einer zwischen 0,1 bis 800 mm /min. einstellbaren Prüfgeschwindigkeit und einer Software: testControl eingesetzt. Es wird ein Druckstempel mit schraubbaren Einsätzen und einem Zylinder (Durchmesser 10 mm), ein Kraftaufnehmer, Fmax. 1 kN, Durchmesser 8 mm, Klasse 0.5 ab 10 N, Klasse 1 ab 2 N nach ISO 7500-1, mit Hersteller-Prüfzertifikat M nach DIN 55350-18 (Zwick-Bruttokraft Fmax 1,45 kN) zur Messung eingesetzt (alles Apparaturen der Firma Zwick GmbH & Co. KG, Ulm, Deutschland) mit der Bestell-Nr. BTC-FR 2.5 TH. D09 für die Prüfmaschine, der Bestell-Nr. BTC-LC 0050N. P01 für den Kraftaufnehmer, der Bestell-Nr. BO 70000 S06 für die Zentriervorrichtung.

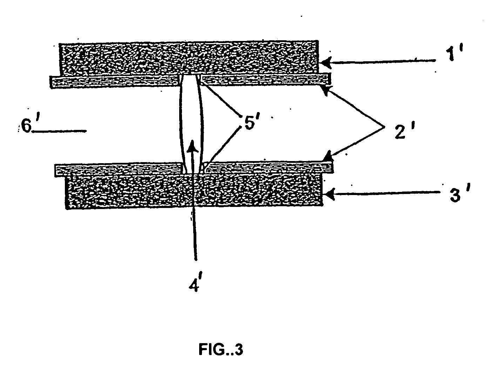

**Figur 3** zeigt die Messung der Bruchfestigkeit einer Tablette, insbesondere die dafür eingesetzte Justierungsvorrichtung (6') der Tablette (4') vor und während der Messung. Darzu wird die Tablette (4') zwischen der oberen Druckplatte (1') und der unteren Druckplatte (3') der nicht dargestellten Vorrichtung zur Kraftaufbringung mit Hilfe von zwei 2-teiligen Einspannvorrichtungen, die jeweils mit der oberen bzw. unteren Druckplatte nach Einstellung des zur Aufnahme und zur Zentrierung der zu messenden Tablette notwendigen Abstands (5') fest verbunden (nicht dargestellt) werden. Zur Einstellung des Abstands (5') können die 2-teiligen Einspannvorrichtungen jeweils auf der Druckplatte, auf der sie gelagert sind, horizontal nach außen oder innen bewegt werden.

Als bruchfest bei einer bestimmten Krafteinwirkung werden auch die Tabletten eingestuft, bei denen kein Bruch feststellbar , aber ggf. eine plastische Verformung der Tablette durch die Krafteinwirkung erfolgt ist.

Bei den erfindungsgemäß erhaltenen Darreichungsformen wird die Bruchfestigkeit nach der aufgeführten Meßmethode bestimmt, wobei von Tabletten abweichenden Darreichungsformen ebenso geprüft werden.

Im Folgenden wird die Erfindung anhand von Beispielen erläutert. Diese Erläuterungen sind lediglich beispielhaft und schränken den allgemeinen Erfindungsgedanken nicht ein.

### Beispiel 1

| Komponenten | Pro Tablette | Gesamtansatz |
|---|---|---|
| Tramadol HCl | 150,0 mg | 314 g |
| Polyethylenoxid, NF, MG 7 000 000 (Polyox WSR 303, Dow Chemicals) | 108,8 mg | 228 g |
| Hydroypropylmethylcellulose (Metholose 90 SH 100 000) | 11,2 mg | 24 g |
| Polyethylenglykol (PEG 6000) | 13,7 mg | 29 g |
| Butylhyxdroxytoluol | 2,6 mg | 5 g |
| Gesamtgewicht | 286,3 mg | 600 g |

Die Komponenten wurden in einem Freifallmischer 15 Minuten lang gemischt, bevor die Pulvermischung in einen Planetwalzen-Extruder mit vier Planetspindeln vom Typ BCG 10 der Fa. LBB Bohle (Ennigerloh, Deutschland) dosiert wurde. Die Dosierung erfolgte gravimetrisch mit 3,0 kg pro Stunde. Der Extruder war mit einer Extrusionsdüse mit einem Durchmesser von 8 mm ausgerüstet. Die Extrusion wurde mit einer Drehzahl von 28,6 UpM bei einer Produkttemperatur von ca. 88 °C durchgeführt.

Nach Abkühlung des extrudierten Stranges auf Raumtemperatur wurde dieser in Scheiben geschnitten, deren Gewicht jeweils dem der endgeformten Tablette entsprach. Die Umformung der Scheiben zu Tabletten erfolgte in einer Ekzenterpresse der Firma Korsch vom Typ EKO mit Hilfe eines Tablettierwerkzeuges umfassend einen runden Stempel mit einem Durchmesser von 10 mm und einen Wölbungsradius von 8 mm.

Die Bruchfestigkeit der Tabletten wurde, wie in der vorliegenden Anmeldung angegeben, bestimmt. Bei einer Krafteinwirkung von 500 N trat kein Bruch der Tabletten auf. Die Tabletten konnten weder mit einem Hammer noch mit Hilfe von Mörser und Pistill zerkleinert werden.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37 °C und die Umdrehungsgeschwindigkeit des Rührers 75min⁻¹. Als Freisetzungsmedium wurden 600 ml Darmsaft pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 21 % |
| 240 min | 86 % |
| 480 min | 93 % |
| 720 min | 91 % |

### Beispiel 2

| Komponenten | Pro Tablette | Gesamtansatz |
|---|---|---|
| Tramadol HCl | 100,0 mg | 314 g |
| Polyethylenoxid, NF, MG 7 000 000 (Polyox WSR 303, Dow Chemicals) | 72,5 mg | 228 g |
| Hydroypropylmethylcellulose (Metholose 90 SH 100 000) | 7,5 mg | 24 g |
| Polyethylenglykol (PEG 6000) | 9,1 mg | 29 g |
| Butylhyxdroxytoluol | 1,7 mg | 5 g |
| Gesamtgewicht | 183,3 mg | 600 g |

Die Komponenten wurden in einem Freifallmischer 15 Minuten lang gemischt, bevor die Pulvermischung in einen Planetwalzen-Extruder mit vier Plantspindeln vom Typ BCG 10 der Fa. LBB Bohle (Ennigerloh, Deutschland) dosiert wurde. Der Extruder war mit einer Extrusionsdüse mit 60 Bohrungen jeweils mit einem Durchmesser von 1 mm ausgerüstet. Die Dosierung erfolgte gravimetrisch mit 3 kg pro Stunde. Die Extrusion wurde mit einer Drehzahl von 28,6 UpM bei einer Produkttemperatur von ca. 88 °C durchgeführt.

Nach Abkühlung der extrudierten Stränge auf Raumtemperatur wurden diese in 1 mm lange, zylindrische Pellets geschnitten, die in Kapseln abgefüllt wurden.

Die Bruchfestigkeit der Pellets wurde nach der in der vorliegenden Anmeldung angegebenen Methode bestimmt. Bei der Krafteinwirkung von 500 N trat kein Bruch auf. Die Pellets konnten weder mit einem Hammer noch mit Hilfe von Mörser und Pistill zerkleinert werden.

Die in vitro Freisetzung des Wirkstoffs aus der Zubereitung wurde in der Blattrührerapparatur mit Sinker nach Pharm Eur. bestimmt. Die Temperatur des Freisetzungsmediums betrug 37 °C und die Umdrehungsgeschwindigkeit des Rührers 75min⁻¹. Als Freisetzungsmedium wurden 600 ml Darmsaft pH 6,8 verwendet. Die jeweils zu einem Zeitpunkt im Lösungsmedium freigesetzte Menge an Wirkstoff wurde spektralphotometrisch bestimmt.

| Zeit | Freigesetzte Wirkstoffmenge |
|---|---|
| 30 min | 58 % |
| 60 min | 81 % |
| 240 min | 96 % |
| 480 min | 99 % |

## Patentansprüche

1. Verfahren zur Herstellung einer gegen Missbrauch gesicherten, festen Darreichungsform mit einer Bruchfestigkeit von mindestens 500 N enthaltend wenigstens einen Wirkstoff mit Missbrauchspotential und wenigstens ein synthetisches und/oder natürliches Polymer (C), **dadurch gekennzeichnet, dass** eine Mischung umfassend den Wirkstoff und die Polymerkomponente (C) mit Hilfe eines Planetwalzen-Extruders durch Schmelzextrusion zu Formlingen verarbeitet wird, die ggf. einer Vereinzelung und Gestaltung zur Darreichungsform unterworfen werden.

2. Verfahren gemäß Anspruch 1, **dadurch gekennzeichnet, dass** als Wirkstoff wenigstens ein Wirkstoff ausgewählt aus der Gruppe umfassend Opioide, Stimulantien, weitere Betäubungsmittel, deren physiologisch verträgliche Verbindungen, deren physiologisch verträgliche Derivate, deren physiologisch verträgliche Racemate, Enantiomere oder Stereoisomere in jeglicher Mischung und deren einsprechende Derivate oder Verbindungen eingesetzt werden.

3. Verfahren gemäß Anspruch 2, **dadurch gekennzeichnet, dass** als physiologisch verträgliche Verbindungen Salze, vorzugsweise Hydrochloride oder Sulfate und als Derivate Ester, Ether oder Amide der Wirkstoffe eingesetzt werden.

4. Verfahren gemäß einem der Ansprüche 1 bis 3, **dadurch gekennzeichnet, dass** als Wirkstoff ein Opioid ausgewählt aus der Gruppe umfassend Oxycodon, Morphin, Hydromorphon, Tramadol und deren physiologisch verträglichen Salze, vorzugsweise Hydrochloride, Sulfate, Saccharinate, deren Solvate deren Stereoisomere, deren Enantiomeren, deren Diastereomeren in beliebigen Mischungen eingesetzt werden.

5. Verfahren gemäß einem Ansprüche 1 bis 3, **dadurch gekennzeichnet, dass** als Wirkstoff wenigstens ein Opioid ausgewählt aus der Gruppe umfassend (2R, 3R)-1-Dimethylamino-3-(3-methoxy-phenyl)-2-methyl-pentan-3-ol, (1RS, 3RS, 6RS)-6-Dimethylaminomethyl-1-(3-methoxy-phenyl)-cyclohexane-1,3-diol, (1 R, 2R)-3-(2-Dimethylaminonethyl-cyclohexyl)-phenol, (1R, 2R)-3-(3-Dimethylamino-1-ethyl-2-methyl-propyl)-phenol, deren physiologisch verträglichen Salze, vorzugsweise Hydrochloride, physiologisch verträgliche Enantiomeren, Stereoisomeren, Diastereomeren und Racemate und deren physiologisch verträglichen Derivate, vorzugsweise Ether, Ester oder Amide eingesetzt werden.

6. Verfahren gemäß einem der Ansprüche 1 bis 5, **dadurch gekennzeichnet, dass** die Polymerkomponente (C) in einer Menge von wenigstens 20 Gew.%, vorzugsweise in einer Menge von 35 bis 99,9 Gew.%, besonders bevorzugt in einer Menge von wenigstens 50 Gew.%, ganz besonders bevorzugt von wenigstens 60 Gew.%, bezogen auf das Gesamtgewicht der Darreichungsform, zum Einsatz kommt.

7. Verfahren gemäß einem der Ansprüche 1 bis 6, **dadurch gekennzeichnet, dass** als Polymer (C) wenigstens ein Polymer ausgewählt aus der Gruppe umfassend Polyalkylenoxide, Polyethylene, Polypropylene, Polyvinylchloride, Polycarbonate, Polystyrrole, Poly(meth)acrylate und deren Copolymerisate und Mischungen aus wenigstens zwei Vertretern der genannten Polymerklassen oder Polymeren eingesetzt wird.

8. Verfahren gemäß Anspruch 7, **dadurch gekennzeichnet, dass** das Polyalkylenoxid ein Polymethylenoxid, Polyethylenoxid und/oder Polypropylenoxid ist.

9. Verfahren gemäß Anspruch 7, **dadurch gekennzeichnet, dass** als Polyalkylenoxid ein Polyethylenoxid mit einem Molekulargewicht von wenigstens 0,5 Mio. eingesetzt wird.

10. Verfahren gemäß Anspruch 9, **dadurch gekennzeichnet, dass** als Polyethylenoxid ein Polyethylenoxid mit einem Molekulargewicht von wenigstens 1 Mio., vorzugsweise von 1 bis 15 Mio. eingesetzt wird.

11. Verfahren gemäß einem der Ansprüche 1 bis 10, **dadurch gekennzeichnet, dass** die Mischung noch weitere Hilfsstoffe (B) und/oder wenigstens ein Wachs (D), enthält.

12. Verfahren gemäß Anspruche 11, **dadurch gekennzeichnet, dass** das Wachs (D) Canaubawachs oder Bienenwachs eingesetzt wird.

13. Verfahren gemäß einem der Ansprüche 1 bis 12, **dadurch gekennzeichnet, dass** die Mischung die Komponente (C) und die ggf. vorhandende Komponente (D) in einer solchen Menge enthält, dass eine Darreichungsform mit einer Bruchfestigkeit von mindestens 500 N erhalten wird.

14. Verfahren gemäß Anspruch 11, **dadurch gekennzeichnet, dass** als Hitfsstoffe (B) Antioxidantien und/oder Weichmacher eingesetzt werden.

15. Verfahren gemäß einem der Ansprüche 1 bis 14, **dadurch gekennzeichnet, dass** ein Planetwalzen-Extruder mit wenigstens drei Planetspindeln eingesetzt wird.

16. Verfahren gemäß einem der Ansprüche 1 bis 15, **dadurch gekennzeichnet, dass** ein Planetwalzen-Extruder mit einer Einzugsschnecke und einer Zentralspindel mit den Planetspindeln eingesetzt wird.

17. Verfahren gemäß einem der Ansprüche 1 bis 16, **dadurch gekennzeichnet, dass** die Mischung im Planetwalzen-Extruder mindestens bis zur Erweichung der Komponente (C) erwärmt, kompaktiert und extrudiert wird.

18. Verfahren gemäß einem der Ansprüche 1 bis 17, **dadurch gekennzeichnet, dass** das plastische Extrudat endgeformt und ggf. vereinzelt wird.

19. Verfahren gemäß einem der Ansprüche 1 bis 18, **dadurch gekennzeichnet, dass** das Extrudat zu Tabletten geformt wird.

20. Verfahren gemäß Anspruch 19, **dadurch gekennzeichnet, dass** die Tabletten mit Hilfe von zwei gegenläufig angetriebenen Walzen mit einander gegenüberliegenden Vertiefungen im Walzenmantel, deren Ausführung die Tablettenform bestimmt, unter Verpressung geformt werden.

21. Verfahren gemäß Anspruch 19, **dadurch gekennzeichnet, dass** die vereinzelten Extrudate jeweils mit Hilfe einer Matrize und mindestens einem formgebenden Stempel zu Tabletten geformt werden.

22. Verfahren gemäß einem der Ansprüche 1 bis 18, **dadurch gekennzeichnet, dass** das Extrudat zu einer multipartikulären Form, vorzugsweise zu Pellets, Spheroiden, Granulaten, vorzugsweise zur zylinderförmigen Granulaten, vereinzelt und ggf. geformt und ggf. zu Tabletten verpresst oder in Kapseln abgefüllt wird.

23. Gegen Missbrauch geschützte Darreichungsform erhältlich nach einem Verfahren gemäß einem der Ansprüche 1 bis 22.

## Claims

1. A process for the production of an abuse-proofed solid dosage form with a breaking strength of at least 500 N containing at least one active ingredient with potential for abuse and at least one synthetic or natural polymer (C), **characterised in that** a mixture comprising the active ingredient and the polymer component (C) is processed by melt extrusion with the assistance of a planetary-gear extruder into formed articles which are optionally subjected to singulation and shaping to yield the dosage form.

2. A process according to claim 1, **characterised in that** the active ingredient used is at least one active ingredient selected from the group comprising opioids, stimulants, further narcotics, the physiologically acceptable compounds thereof, the physiologically acceptable derivatives thereof, the physiologically acceptable racemates, enantiomers or stereoisomers thereof in any mixture and the corresponding derivatives or compounds thereof.

3. A process according to claim 2, **characterised in that** the physiologically acceptable compounds used are salts, preferably hydrochlorides or sulfates, and the derivatives used are esters, ethers or amides of the active ingredients.

4. A process according to any one of claims 1 to 3, **characterised in that** the active ingredient used is an opioid selected from the group comprising oxycodone, morphine, hydromorphone, tramadol and the physiologically acceptable salts thereof, preferably hydrochlorides, sulfates, saccharinates, the solvates thereof, stereoisomers thereof, enantiomers thereof, diastereomers thereof in any desired mixtures.

5. A process according to any one of claims 1 to 3, **characterised in that** the opioid used is at least one opioid selected from the group comprising (2R,3R)-1-dimethylamino-3-(3-methoxyphenyl)-2-methylpentan-3-ol, (1RS,3RS,6RS)-6-dimethylaminomethyl-1-(3-methoxyphenyl)-cyclohexane-1,3-diol, (1R,2R)-3-(2-dimethylaminomethylcyclohexyl)phenol, (1R,2R)-3-(3-dimethylamino-1-ethyl-2-methylpropyl)phenol, the physiologically acceptable salts thereof, preferably hydrochlorides, physiologically acceptable enantiomers, stereoisomers, diastereomers and racemates and the physiologically acceptable derivatives thereof, preferably ethers, esters or amides.

6. A process according to any one of claims 1 to 5, **characterised in that** polymer component (C) is used in a quantity of at least 20 wt.%, preferably in a quantity of 35 to 99.9 wt.%, particularly preferably in a quantity of at least 50 wt.%, very particularly preferably in a quantity of at least 60 wt.%, relative to the total weight of the dosage form.

7. A process according to any one of claims 1 to 6, **characterised in that** the polymer (C) used is at least one polymer selected from the group comprising polyalkylene oxides, polyethylenes, polypropylenes, polyvinyl chlorides, polycarbonates, polystyrenes, poly(meth)acrylates and the copolymers thereof and mixtures of at least two of the stated polymers or classes of polymers.

8. A process according to claim 7, **characterised in that** the polyalkylene oxide is a polymethylene oxide, polyethylene oxide and/or polypropylene oxide.

9. A process according to claim 7, **characterised in that** the polyalkylene oxide used is a polyethylene oxide with a molecular weight of at least 0.5 million.

10. A process according to claim 9, **characterised in that** the polyethylene oxide used is a polyethylene oxide with a molecular weight of at least 1 million, preferably of 1 to 15 million.

11. A process according to any one of claims 1 to 10, **characterised in that** the mixture additionally contains further auxiliary substances (B) and/or at least one wax (D).

12. A process according to claim 11, **characterised in that** the wax used is carnauba wax or beeswax.

13. A process according to any one of claims 1 to 12, **characterised in that** the mixture contains component (C) and the optionally present component (D) in a quantity such that a dosage form with a breaking strength of at least 500 N is obtained.

14. A process according to claim 11, **characterised in that** antioxidants and/or plasticisers are used as auxiliary substances (B).

15. A process according to any one of claims 1 to 14, **characterised in that** a planetary-gear extruder with at least three planetary spindles is used.

16. A process according to any one of claims 1 to 15, **characterised in that** a planetary-gear extruder with a feed screw and a central spindle with the planetary spindles is used.

17. A process according to any one of claims 1 to 16, **characterised in that** the mixture in the planetary-gear extruder is heated at least until component (C) has softened, is compacted and extruded.

18. A process according to any one of claims 1 to 17, **characterised in that** the plastic extrudate is finally formed and optionally singulated.

19. A process according to any one of claims 1 to 18, **characterised in that** the extrudate is formed into tablets.

20. A process according to claim 19, **characterised in that** the tablets are formed with press moulding with the assistance of two contrarotating rolls with mutually opposing recesses in the roll sleeve, the construction of which recesses determines the tablet shape.

21. A process according to claim 19, **characterised in that** the singulated extrudates are in each case formed into tablets with the assistance of a die and at least one shaping punch.

22. A process according to any one of claims 1 to 18, **characterised in that** the extrudate is singulated into a multiparticulate form, preferably pellets, spheroids, granules, preferably cylindrical granules, and optionally formed and optionally press-moulded into tablets or packaged in capsules.

23. An abuse-proofed dosage form obtainable by a process according to any one of claims 1 to 22.

## Revendications

1. Procédé pour la fabrication d'une forme galénique solide protégée contre un usage détourné, ayant une résistance à la rupture d'au moins 500 N, contenant au moins une substance active à potentiel d'usage détourné et au moins un polymère (C) naturel et/ou synthétique, **caractérisé en ce qu'**on transforme un mélange comprenant la substance active et le composant polymère (C), à l'aide d'une extrudeuse à cylindres planétaires par extrusion de masse fondue, en ébauches qui sont éventuellement soumises à un isolement et un façonnage en la forme galénique.

2. Procédé selon la revendication 1, **caractérisé en ce qu'**on utilise comme substance active au moins une substance active choisie dans le groupe comprenant des opioïdes, des stimulants, d'autres narcotiques, leurs composés physiologiquement acceptables, leurs dérivés physiologiquement acceptables, leurs racémates, énantiomères ou stéréoisomères physiologiquement acceptables en mélange quelconque et leurs dérivés ou composés correspondants.

3. Procédé selon la revendication 2, **caractérisé en ce qu'**on utilise comme composés physiologiquement acceptables des sels, de préférence des chlorhydrates ou sulfates et comme dérivés des esters, éthers ou amides des substances actives.

4. Procédé selon l'une quelconque des revendications 1 à 3, **caractérisé en ce qu'**on utilise comme substance active un opioïde choisi dans le groupe comprenant l'oxycodone, la morphine, l'hydromorphone, le tramadol et leurs sels physiologiquement acceptables, de préférence chlorhydrates, sulfates, saccharinates, leurs produits de solvatation, leurs stéréoisomères, leurs énantiomères, leurs diastéréoisomères en mélanges quelconques.

5. Procédé selon l'une quelconque des revendications 1 à 3, **caractérisé en ce qu'**on utilise comme substance active au moins un opioïde choisi dans le groupe comprenant le (2R,3R)-1-diméthylamino-3-(3-méthoxy-phényl)-7-méthyl-pentan-3-ol, le (1RS,3RS,6RS)-6-diméthylaminométhyl-1-(3-méthoxy-phényl)-cyclohexane-1,3-diol, le (1R,2R)-3-(2-diméthylaminométhyl-cyclohexyl)-phénol, le (1R,2R)-3-(3-diméthylamino-1-éthyl-2-méthyl-propyl)-phénol, leurs sels physiologiquement acceptables, de préférence les chlorhydrates, leurs énantiomères, stéréoisomères, diastéréoisomères et racémates physiologiquement acceptables et leurs dérivés, de préférence éthers, esters ou amides, physiologiquement acceptables.

6. Procédé selon l'une quelconque des revendications 1 à 5, **caractérisé en ce que** le composant polymère (C) est utilisé en une quantité d'au moins 20 % en poids, de préférence en une quantité de 35 à 99,9 % en poids, de façon particulièrement préférée en une quantité d'au moins 50 % en poids, de façon tout particulièrement préférée d'au moins 60 % en poids, par rapport au poids total de la forme galénique.

7. Procédé selon l'une quelconque des revendications 1 à 6, **caractérisé en ce qu'**on utilise comme polymère (C) au moins un polymère choisi dans le groupe comprenant des polyoxyalkylènes, polyéthylènes, polypropylènes, poly(chlorure de vinyle)s, polycarbonates, polystyrènes, poly(méth)acrylates et leurs copolymérisats et des mélanges d'au moins deux représentants des polymères nommés ou classes de polymères nommées.

8. Procédé selon la revendication 7, **caractérisé en ce que** le polyoxyalkylène est un polyoxyméthylène, polyoxyéthylène et/ou polyoxypropylène.

9. Procédé selon la revendication 7, **caractérisé en ce qu'**on utilise comme polyoxyalkylène un polyoxyéthylène ayant une masse moléculaire d'au moins 0,5 million.

10. Procédé selon la revendication 9, **caractérisé en ce qu'**on utilise comme polyoxyéthylène un polyoxyéthylène ayant une masse moléculaire d'au moins 1 million, de préférence de 1 à 15 millions.

11. Procédé selon l'une quelconque des revendications 1 à 10, **caractérisé en ce que** le mélange contient encore d'autres adjuvants (B) et/ou au moins une cire (D).

12. Procédé selon la revendication 11, **caractérisé en ce qu'**on utilise comme cire (D) la cire de carnauba ou la cire d'abeille.

13. Procédé selon l'une quelconque des revendications 1 à 12, **caractérisé en ce que** le mélange contient en une quantité telle le composant (C) et le composant (D) éventuellement présent qu'on obtient une forme galénique ayant une résistance à la rupture d'au moins 500 N.

14. Procédé selon la revendication 11, **caractérisé en ce qu'**on utilise comme adjuvants (B) des antioxydants et/ou des plastifiants.

15. Procédé selon l'une quelconque des revendications 1 à 14, **caractérisé en ce qu'**on utilise une extrudeuse à cylindres planétaires comportant au moins trois axes planétaires.

16. Procédé selon l'une quelconque des revendications 1 à 15, **caractérisé en ce qu'**on utilise une extrudeuse à cylindres planétaires comportant une vis d'alimentation et un axe central avec les axes planétaires.

17. Procédé selon l'une quelconque des revendications 1 à 16, **caractérisé en ce que** dans l'extrudeuse à cylindres planétaires le mélange est chauffé au moins jusqu'au ramollissement du composant (C), compacté et extrudé.

18. Procédé selon l'une quelconque des revendications 1 à 17, **caractérisé en ce que** l'extrudat plastique est mis sous la forme finale et éventuellement isolé.

19. Procédé selon l'une quelconque des revendications 1 à 18, **caractérisé en ce que** l'extrudat est mis sous forme de comprimés.

20. Procédé selon la revendication 19, **caractérisé en ce que** les comprimés sont mis en forme par pressage à l'aide de deux cylindres contrarotatifs à cavités opposées les unes aux autres dans l'enveloppe des cylindres, dont la conception détermine la forme des comprimés.

21. Procédé selon la revendication 19, **caractérisé en ce que** les extrudats isolés sont chacun mis en forme de comprimés à l'aide d'une matrice et d'au moins un poinçon donnant la forme.

22. Procédé selon l'une quelconque des revendications 1 à 18, **caractérisé en ce que** l'extrudat est isolé en une forme multiparticulaire, de préférence en granules, sphéroïdes, granulés, de préférence en les granulés cylindriques, et éventuellement façonnés et éventuellement pressés en comprimés ou introduits dans des capsules.

23. Forme galénique protégée contre un usage détourné, pouvant être obtenue conformément à un procédé selon l'une quelconque des revendications 1 à 22.
